# EUROPÄISCHE PATENTSCHRIFT

(11) **EP 3 273 897 B1**
(45) Veröffentlichungstag und Bekanntmachung des Hinweises auf die Patenterteilung: **16.10.2024**
(21) Anmeldenummer: 16711825.6
(22) Anmeldetag: 23.03.2016
(51) Int. Cl.: A61B 90/50, A61B 34/00, A61B 17/00

(54) **VERFAHREN UND VORRICHTUNG ZUM STEUERN EINES CHIRURGISCHEN MECHATRONISCHEN ASSISTENZSYSTEMS MITTELS EINES HALTEARMS FÜR MEDIZINISCHE ZWECKE**
METHOD AND DEVICE FOR CONTROLLING A SURGICAL MECHATRONIC ASSISTANCE SYSTEM BY MEANS OF A HOLDING ARM FOR MEDICAL PURPOSES
PROCÉDÉ ET DISPOSITIF POUR ASSURER LA COMMANDE D'UN SYSTÈME D'ASSISTANCE MÉCATRONIQUE CHIRURGICAL AU MOYEN D'UN BRAS DE RETENUE À USAGE MÉDICAL

(30) Priorität: 27.03.2015 DE 102015104810
(43) Veröffentlichungstag der Anmeldung: 31.01.2018
(73) Patentinhaber: Brainlab Robotics GmbH, 81829 München (DE)
(72) Erfinder: NOWATSCHIN, Stephan, 81677 München (DE); KRINNINGER, Maximilian, 82234 Weßling-Oberpfaffenhofen (DE); GIERLACH, Dominikus, 80798 München (DE)
(74) Vertreter: Eisenführ Speiser
(86) Internationale Anmeldenummer: PCT/EP2016/056459
(87) Internationale Veröffentlichungsnummer: WO 2016/156168

(56) Entgegenhaltungen:
- EP-A1- 1 557 134
- EP-A1- 2 047 805
- EP-A2- 1 520 548
- WO-A1-2011/085815
- WO-A2-2014/151550
- US-A1- 2005 209 614
- US-A1- 2007 129 846
- US-A1- 2014 222 023

## Beschreibung

Die Erfindung betrifft einen Haltearm für medizinische Zwecke, insbesondere zum Halten eines chirurgischen mechatronischen Assistenzsystems mit einem proximalen Ende zum Befestigen des Haltearms an einer Basis und einem distalen Ende zum Aufnehmen eines chirurgischen mechatronischen Assistenzsystems. Ferner betrifft die Erfindung ein Verfahren zum Steuern eines an einem Haltearm gekoppelten mechatronischen Assistenzsystems, unter Verwendung eines derartigen Haltearms.

Haltearme der eingangs genannten Art sind bereits seit längerem bekannt und werden in der Chirurgie insbesondere dazu eingesetzt, einen Operateur von statischer Haltearbeit zu entlasten. Ein derartiger Haltearm wird eingesetzt, um ein mechatronisches Assistenz-system und/oder ein chirurgisches Instrument zu halten, wie etwa einen Manipulator, ein Endoskop, eine chirurgische Klemme und dergleichen. Insbesondere zum Halten von Endoskopen haben sich die eingangs genannten Haltearme bewährt. Bei der endoskopischen Chirurgie bedient ein Operateur in der Regel mit beiden Händen ein Instrument, während ein Assistent das Endoskop hält, um das Operationsfeld über einen Bildschirm sichtbar zu machen. Das Halten des Endoskops über einen längeren Zeitraum, ist sehr ermüdend. Aus diesem Grund werden vermehrt Haltearme eingesetzt.

Eine solcher Haltearm und sein solches Verfahren sind beispielsweise in der Patentanmeldung mit dem Aktenzeichen DE 10 2014 016 823.9 beschrieben. Es ist ein Haltearm mit einer Arm-Bedieneinrichtung beschrieben, die zum Verbringen des Haltearms in eine gewünschte Pose eingerichtet ist und die weiterhin eingerichtet ist bei Kontakt zwischen einer Bedienperson und einem der ersten und zweiten Armsegmente das zugeordnete Gelenk freizugeben.

Aus US 2014/0222023 A1 sind ein Verfahren und ein System zum robotischgestützten Durchführen von chirurgischen Verfahren bekannt. Das System umfasst einen robotischen Arm mit einem Endeffektor und einem hybriden Steuermechanismus für die robotische Chirurgie.

EP 1 520 548 A2 offenbart eine medizinische Stützvorrichtung. Die Stützvorrichtung kann zwischen einem bewegbaren und einem verriegelten Zustand geschaltet werden. Hierzu ist am Endeffektor ein Druckschalter vorgesehen.

Weiterhin sind aus EP 1 557 134 A1 ein Verfahren und eine Vorrichtung zum Halten eines medizinischen Geräts bekannt. Die Vorrichtung umfasst einen Arm mit einer Bestimmungseinheit, die bestimmt, ob ein Bediener an dem Arm eine unzulässige Handlung vornimmt. Ist dies der Fall, wird der Arm gesperrt.

EP 2 047 805 A1 offenbart ein robotisches System für die minimalinvasive Chirurgie, welches ein chirurgisches Instrument in Antwort auf Befehle eines Chirurgen positionieren kann. Das System ist in der Lage, ohne Kalibrierung eine Bewegung zu berechnen, die der Roboter ausführen muss, um mit dem chirurgischen Instrument einen bestimmten Ort anzufahren.

Aus US 2005/0209614 A1 ist eine Vorrichtung für die Anostomoseoperation bekannt. Die Vorrichtung hat einen Haltearm und einen Operationsstab. An dem Operationsstab ist ein Bedienknopf angebracht, über den sich die Gelenke des Haltearms freigeben lassen.

US 2007/0129846 A1 offenbart einen anthropomorphen medizintechnischen Roboterarm mit einem proximalen Basisende und einem distalen Funktionsende, wobei der Arm ein Basisgelenk, ein Mittelgelenk und ein Distalgelenk aufweist, sowie ein erstes Armelement zwischen Basisgelenk und Mittelgelenk und ein zweites Armelement zwischen Mittelgelenk und Distalgelenk, wobei zu- und/oder abschaltbare, auf die Gelenke wirkende Bewegungshemmer vorgesehen sind, welche den funktionell möglichen Arbeitsbereich des Roboterarms durch eine Hemmung der Bewegungsfreiheit der Gelenke auf den möglichen Bewegungsbereich eines menschlichen Arms einschränken. Sie betrifft ferner ein Verfahren zum Betrieb eines solchen Roboterarms.

Aus WO 2011/085815 A1 ist ferner ein Steuersystem für ein chirurgisches Navigationssystem bekannt. Eine Einheit erkennt Nutzereingaben und speist diese in das Navigationssystem ein.

Schließlich offenbart WO 2014/151550 A2 ein chirurgisches System mit einem Handgerät, welches sich über Knöpfe steuern lässt. Ferner umfasst das System auch einen Werkzeugpfaderzeuger, der einen Pfad für das chirurgische Werkzeug erzeugen kann.

Es ist es eine Aufgabe der vorliegenden Erfindung, einen Haltearm sowie ein Verfahren anzugeben, deren Einsatzbereich erweitert ist.

Bezüglich des Haltearms wird diese Aufgabe durch einen Haltearm für medizinische Zwecke mit den Merkmalen des Anspruchs 1 gelöst. Insbesondere durch einen Haltearm zum Halten eines mechatronischen Assistenzsystems, mit einem proximalen Ende zum Befestigen des Haltearms an einer Basis und einem distalen Ende zum Aufnehmen des chirurgischen mechatronischen Assistenzsystems; einer Assistenz-Schnittstelle an dem distalen Ende zum Koppeln des Haltearms mit dem Assistenzsystem zum Steuern des Assistenzsystems; und einer Assistenz-Bedieneinrichtung, die dazu eingerichtet, eine auf ein Steuern des Assistenzsystems gerichtete Bedienaktion eines Bedieners zu erfassen und bedarfsweise ein Übertragen eines die Bedienaktion repräsentierenden Anforderungssignals an die Assistenz-Schnittstelle zwecks Steuerung des chirurgischen mechatronischen Assistenzsystems zu bewirken.

Vorteilhafterweise ist nun die Grundlage geschaffen, um mittels einer Bedienaktion, einen Stellantrieb eines an die Assistenz-Schnittstelle angeschlossenen mechatronisches Assistenzsystems zu steuern. Als Assistenzsysteme in dem erfindungsgemäßen Sinn werden jegliche Art von mechatronischen Manipulatoren, die in der Chirurgie eingesetzt werden, verstanden, so wie insbesondere Endoskope, Exoskope, Laparoskope, Trokare und dergleichen. Die Assistenz-Schnittstelle am distalen Ende des Haltearms ist dazu ausgebildet, sowohl mechanisch mit dem Assistenzsystem zu koppeln, um dieses in einer definierten Position zum Haltearm zu halten, als auch die notwendigen weiteren Anschlüsse, wie insbesondere einen Anschluss für elektrische Energie und einen Anschluss zum Übertragen von Signalen, insbesondere Anforderungssignalen, bereitzustellen.

Die Assistenz-Bedieneinrichtung kann verschiedentlich ausgebildet sein. Die Assistenz-Bedieneinrichtung weist Kontaktmittel auf, die dazu vorgesehen sind, dass ein Bediener mit diesen in Kontakt kommt. In einer offenbarten Alternative ist ein Kontaktmittel als berührungsempfindlicher Sensor, bevorzugt als berührungsempfindliches Sensorfeld ausgebildet. Derart kann ein Bediener beispielsweise mit dem Finger Bewegungspfade abfahren, die als zeitlicher Verlauf von Koordinaten erfasst und von einer Verarbeitungseinheit des Haltearms weiterverarbeitet werden können. Somit kann beispielsweise eine kontaktbasierte Gestensteuerung eines an die Assistenz-Schnittstelle angeschlossenen mechatronisches Assistenzsystems realisiert werden. Die Assistenz-Bedieneinrichtung kann dazu eingerichtet sein ein angeschlossenes mechatronisches Assistenzsystems proportional zu einer Bedienaktion in Form eines Bewegungspfades anzusteuern. So kann die Assistenz-Bedieneinrichtung beispielsweise dazu eingerichtet sein bei einer geradlinigen Fingerbewegung, z.B. von 1cm, ein angeschlossenes Endoskop (mechatronisches Assistenzsystem) 1cm auszufahren. Es soll verstanden werden, dass eine derartige Ansteuerung durch einen Proportionalitätsfaktor "übersetzt" werden kann, so dass bei einer geradlinigen Fingerbewegung, z.B. von 1cm, ein angeschlossenes Endoskop 2 cm ausfährt oder umgekehrt.

Das Kontaktmittel kann in einer offenbarten Alternative als Taster oder Knopf ausgebildet sein. Ein als Taster oder Knopf ausgebildetes Kontaktmittel kann beispielsweise dazu vorgesehen sein, die Assistenz-Bedieneinrichtung zur Ausgabe eines vordefinierten Anforderungssignals ("Fahre in Endposition"; "Rotation um 90 Grad" etc.) anzusteuern. Die Assistenz-Bedieneinrichtung kann dazu eingerichtet sein zu Ermitteln, welche Art von Assistenzsystem an dem Haltearm angekoppelt ist. Die Assistenz-Bedieneinrichtung ist bevorzugt dazu eingerichtet, die Belegung eines Kontaktmittels mit einer Steuerfunktion in Abhängigkeit der Art des erkannten Assistenzsystems anzupassen.

Alternativ oder zusätzlich weist die Assistenz-Bedieneinrichtung eine Erfassungseinheit für eine kontaktlose Erfassung der Bedienaktion auf. Dies hat unter anderem Vorteile hinsichtlich der Sterilität, da ein Bediener den Halterarm zur Steuerung des Assistenzsystems nicht berühren muss. Die Erfassungseinheit weist bevorzugt eine Kamera und/oder einen Abstandssensor auf. Die Kamera ist beispielsweise als eine CCD-Kamera ausgebildet, der Abstandssensor beispielsweise als ein kapazitiver Sensor. Die oben mit Bezug auf das berührungsempfindliche Sensorfeld beschriebene kontaktbasierte Gestensteuerung kann gleichermaßen als kontaktlose Gestensteuerung mittels der Erfassungseinheit für eine kontaktlose Erfassung der Bedienaktion realisiert sein.

Die Kontaktmittel und die Erfassungseinheit für eine kontaktlose Erfassung der Bedienaktion können redundant vorgesehen sein und die Assistenz-Bedieneinrichtung bevorzugt ausgebildet sein zu Verifizieren, ob tatsächlich eine auf ein Steuern des Assistenzsystems gerichtete Bedienaktion eines Bedieners, insbesondere eine Gesten-Bedienaktion vorliegt.

Gemäß der Erfindung weist die Assistenz-Bedieneinrichtung wenigstens einen Steuerungsring auf. Der Steuerring kann um wenigstens ein Armsegment des Haltearms herum umfänglich und/oder axial beweglich angeordnet sein. Weist der Haltearm mehrere Armsegmente auf, so kann beispielsweise für jedes Armsegment ein Steuerungsring oder eine anderweitig ausgestaltete Assistenz-Bedieneinrichtung vorgesehen sein. Alternativ oder zusätzlich sind an einem Armsegment zwei oder mehr Steuerungsringe vorgesehen.

Der Haltearm weist eine der Assistenz-Bedieneinrichtung zugeordnete Verarbeitungseinheit auf, die dazu eingerichtet ist die Bedienaktion in eine Datenstruktur umzuwandeln. Bevorzugt ist die Verarbeitungseinheit zumindest zeitweise mit dem Kontaktmittel, der Erfassungseinheit und/oder dem Steuerungsring, insbesondere zu Übermittlung von Koordinaten und deren Erfassungszeit, kommunikativ gekoppelt. Eine kommunikative Kopplung kann beispielsweise durch eine serielle Schnittstelle oder dergleichen realisiert sein. Bevorzugt ist jedem Armsegment, an dem eine Assistenz-Bedieneinrichtung angeordnet ist, eine eigene Verarbeitungseinheit zugeordnet.

Die Verarbeitungseinheit ist dazu eingerichtet, die Bedienaktion in eine Datenstruktur umzuwandeln, die einen 3D-Bewegungsvektor und/oder einen Geschwindigkeitsgröße umfasst. Bevorzugt ist die Verarbeitungseinheit dazu eingerichtet, wenigstens zwei zeitlich aufeinander folgende Koordinaten in einen 3D-Bewegungsvektor (Richtungsvektor) umzuwandeln. Ebenfalls ist die Verarbeitungseinheit vorzugsweise dazu eingerichtet, aus den Erfassungszeiten der wenigstens zwei zeitlich aufeinander folgenden Koordinaten eine Bewegungsgeschwindigkeit zu ermitteln. Die Verarbeitungseinheit ist bevorzugt dazu eingerichtet, die Datenstruktur in das Anforderungssignal für das Assistenzsystem umzuwandeln. Alterativ wird die Datenstruktur selbst als Anforderungssignal an das Assistenzsystem weitergeleitet.

Der Haltearm ist mit einer Basis-Schnittstelle an dem proximalen Ende zum Verbinden des Haltearms mit einer externen Steuereinheit zum Übertragen von Signalen an den und von dem Haltearm ausgestattet. Der Haltearm weist eine Übertragungseinrichtung auf, welche innerhalb des Haltearms angeordnet ist und die Assistenz-Schnittstelle mit der Basis-Schnittstelle zum Übertragen Signalen zwischen den Schnittstellen verbindet.

Die Übertragungseinrichtung weist bevorzugt Mittel zum Übertragen von elektrischer Energie auf. Dadurch sind sämtliche Kabel, die zur Übertragung von elektrischer Energie und/oder Daten von der Basis-Schnittstelle zur Assistenz-Schnittstelle erforderlich sind, im Inneren des Haltearms angeordnet und dadurch während des Betriebs des Haltearms geschützt. Zum Übertragen von Daten weisen die Basis-Schnittstelle und/oder die Assistenz-Schnittstelle vorzugsweise Übertragungsmittel zum Übertragen der von dem Sensor oder den Sensoren erfassten Daten auf. Diese Übertragungsmittel umfassen vorzugsweise Schnittstellen, wie insbesondere Bluetooth^{®}, USB, RS-232 oder ähnliche. Bevorzugt weist die Übertragungseinrichtung ein Bussystem auf.

Der Haltearm weist bevorzugt mehrere Armsegmente und mehrere Gelenke auf, mittels derer die Armsegmente gelenkig miteinander verbunden sind. Vorzugsweise weist der Haltearm wenigstens sechs Armsegmente und wenigstens sechs Gelenke auf. Die Armsegmente selbst sind im Wesentlichen starr und vorzugsweise im Wesentlichen stabförmig. Der Begriff "stabförmig" umfasst hier sowohl im Wesentlichen gerade Armsegmente als auch leicht bis stark gekrümmte Armsegmente. Bei einem derartigen Haltearm wechseln sich Armsegmente und Gelenke stets ab. Der Haltearm ist vorzugsweise als sogenannter passiver Haltearm ausgebildet und weist daher ausschließlich aktiv gebremste Gelenke auf, jedoch keine angetriebenen Gelenke, wie dies bei robotischen Haltearmen häufig der Fall ist. Jedes Gelenk ist daher nur freigeb- und arretierbar, allerdings nicht antreibbar. Hierdurch ist der Haltearm einfach aufgebaut und bedarf keiner komplexen Steuerung zu seinem Betrieb. Der Haltearm kann manuell verstellbar sein, indem die einzelnen Gelenke gegen die Bremskraft der Bremsen verstellt werden.

Gemäß einer weiteren bevorzugten Ausgestaltung weist der Haltearm sechs Freiheitsgrade auf. Besonders bevorzugt weist der Haltearm sieben Freiheitsgrade auf. Während sechs Freiheitsgrade ausreichen, um jeden Punkt im Raum zu erreichen, ist es bei sieben Freiheitsgraden möglich, jeden Punkt mit verschiedenen Posen zu erreichen, sodass der Haltearm stets so ausrichtbar ist, dass beispielsweise das Operationsfeld leicht zugänglich ist. Daher ist es besonders bevorzugt, dass der Haltearm sieben Freiheitsgrade aufweist.

Gemäß einer bevorzugten Ausführungsform weist der Haltearm sieben Armsegmente und sieben Gelenke auf, wobei jedem Armsegment ein Gelenk zugeordnet ist. Jedes Gelenk weist gemäß diesem Ausführungsbeispiel vorzugsweise einen Freiheitsgrad auf, sodass der Haltearm insgesamt sieben Freiheitsgrade aufweist. Es ist auch möglich, dass jedes Gelenk zwei oder mehr Freiheitsgrade hat, wobei Gelenke mit einem Freiheitsgrad aufgrund ihrer Stabilität bevorzugt sind. Vorzugsweise sind sämtliche Gelenke als rotatorische Gelenke ausgebildet.

Vorzugsweise sind einige der Gelenke als rotatorische Gelenke und einige als translatorische Gelenke ausgebildet. Vorzugsweise sind die Gelenke, wenn diese sämtlich rotatorisch ausgebildet sind, derart in dem Haltearm angeordnet, dass Achsen von entlang des Haltearms aufeinanderfolgenden Gelenken, vom proximalen zum distalen Ende des Haltearms hin, jeweils senkrecht aufeinander stehen.

Es hat sich als vorteilhaft herausgestellt, wenn die Assistenz-Bedieneinrichtung an oder in wenigstens einem der Armsegmente angeordnet ist. Die Assistenz-Bedieneinrichtung kann über mehrere Armsegmente verteilt angeordnet sein. Die Verarbeitungseinheit ist vorzugsweise zumindest zeitweise mit der Übertragungseinrichtung gekoppelt und dazu eingerichtet den Haltearm betreffende Sensordaten und/oder Arbeitsraumdaten aus der Übertragungseinrichtung auszulesen. Die den Haltearm betreffenden Sensordaten können von Sensoren des Haltearms stammen, beispielsweise von Lagesensoren.

In einer weiteren bevorzugten Ausführungsform weist der Haltearm in wenigstens einem Gelenk einen Lagesensor zum Erfassen einer Stellung des Gelenks auf. Vorzugsweise ist in jedem Gelenk ein Lagesensor zum Erfassen der Stellung eines Gelenks angeordnet. Ein derartiger Lagesensor kann beispielsweise als kapazitiver Wegaufnehmersensor ausgebildet sein, der mechanisch einen Bewegungsweg des Gelenks aufnimmt, und so eine Winkelstellung bestimmt, oder als Beschleunigungssensor, der eine Bewegung des Gelenks im Raum bestimmt. Zusätzlich oder alternativ sind Bewegungssensoren in den Armsegmenten vorgesehen, sodass die Lage der Armsegmente im Raum bestimmbar ist. Hierdurch ist eine Pose des Haltearms bestimmbar, welche wiederum über die Basis-Schnittstelle und/oder die Assistenz-Schnittstelle an das Assistenzsystem und/oder an die externe Steuereinheit bereitgestellt werden kann. Dies ist insbesondere vorteilhaft, wenn ein OP-Navigationssystem verwendet wird und dieses die Informationen über die Pose des Haltearms verwendet, um eine Navigation zu koordinieren. Weiterhin ist über die Pose des Haltearms ermittelbar, ob eine Kollision mit weiteren Geräten oder dem Haltearm selbst droht. Hierdurch ist die Sicherheit des Haltearms weiter verbessert.

Wird ein Beschleunigungssensor als Lagesensor verwendet, ist es auch möglich, eine Bewegung des Haltearms insgesamt, ohne Veränderung der Pose zu detektieren. Wird beispielsweise der Operationstisch während der Operation bewegt, kann der Haltearm diese Bewegung erfassen. Es kann vorgesehen sein, dass der Haltearm bei einer bestimmten Neigung des Operationstisches, beispielsweise ab 15 Grad, dazu eingerichtet ist, ein Warnsignal auszugeben. Wird eine Neigung des Operationstisches zu steil eingestellt, ist es möglich, dass ein Patient auf dem Operationstisch verrutscht, wodurch Verletzungen hervorgerufen werden können. Ist beispielsweise an der Assistenz-Schnittstelle des Haltearms ein Endoskop angeordnet, welches beispielsweise in die Nase eines Patienten eingeführt ist, und wird dann die Neigung des Tisches verstellt, so detektiert der Haltearm mittels der Bewegungssensoren einerseits die Neigung des Tisches, andererseits aufgrund von Drehmomentsensoren in den Gelenken auch eine Belastungsänderung an dem Endoskop, was ebenfalls ein Verrutschen des Patienten auf dem Operationstisch andeuten kann.

In einer weiteren bevorzugten Ausgestaltung ist in wenigstens einem Gelenk ein Drehmomentsensor zum Erfassen eines auf das Gelenk wirkenden Drehmoments angeordnet. Vorzugsweise ist in allen Gelenken ein derartiger Drehmomentsensor angeordnet. Über das Erfassen von auf die Gelenke wirkenden Drehmomenten ist es möglich, eine Kraft, die am distalen Ende des Haltearms wirkt, zu bestimmen. Dadurch ist einerseits ein Gewicht eines Assistenzsystems bestimmbar, welches an dem distalen Ende gekoppelt ist. Andererseits sind auch bei der Verwendung des Haltearms auf diesen wirkende Kräfte bestimmbar. So ist es beispielsweise denkbar, dass an der Assistenz-Schnittstelle ein Endoskop angeordnet ist. Beim Handhaben des Endoskops, beispielsweise Einführen des Endoskops in eine Körperöffnung eines Patienten, kann so ein Widerstand bestimmt werden, auf den das Endoskop stößt. Dadurch ist erkennbar, ob eine Verletzung des Patienten droht. Vorzugsweise werden die erfassten Drehmomentdaten der Verarbeitungseinheit zur Verfügung gestellt.

Es hat sich als vorteilhaft herausgestellt, wenn die Verarbeitungseinheit dazu eingerichtet ist, bei der Umwandlung der Bedienaktion in die Datenstruktur die den Haltearm betreffende Sensordaten zu berücksichtigen. Besonders bevorzugt ist die Verarbeitungseinheit dazu eingerichtet zu Ermitteln, ob das chirurgische mechatronische Assistenzsystem bei Ansteuerung gemäß dem Anforderungssignal innerhalb eines zulässigen Arbeitsraums verbleiben würde. Gemäß einer weiteren bevorzugten Ausgestaltung ist die Verarbeitungseinheit dazu eingerichtet, ein Übertragen des Anforderungssignals an das chirurgische mechatronische Assistenzsystem nur dann zu bewirken, wenn das chirurgische mechatronische Assistenzsystem bei Ansteuerung gemäß der Anforderungssignal innerhalb eines zulässigen Arbeitsraum verbleiben würde.

Ferner kann vorgesehen sein, dass eine zuvor angefahrene Position eines Assistenzsystems, insbesondere eines Endoskops, aus dem zulässigen Arbeitsraum ausgeschlossen wird und dementsprechend nicht mehr angefahren werden darf. Dadurch kann eine Verletzung vermieden werden. Die den Haltearm betreffenden Arbeitsraumdaten können beispielsweise aus einer über die Basis-Schnittstelle angeschlossenen externen Steuereinheit stammen, die Teil eines OP-Navigationssystems. Alternativ oder zusätzlich können die Arbeitsraumdaten durch eine Planungsdatei, DICOM-Datei o.ä. bereitgestellt sein.

Die Bedieneinrichtung kann ein Signalmittel zum Ausgeben eines durch den Bediener wahrnehmbaren Warnsignals aufweisen. Die Verarbeitungseinheit ist vorzugsweise dazu eingerichtet das Signalmittel anzusteuern, wenn das chirurgische mechatronische Assistenzsystem bei Ansteuerung gemäß der Anforderungssignals den zulässigen Arbeitsraum verlassen würde und/oder sich einer Arbeitsraumgrenze bis auf einen vorgegebenen Abstand nähern würde. Das Signalmittel kann als Vibrationsmodul und/oder als optisches Anzeigemodul ausgebildet. Die Verarbeitungseinheit kann eingerichtet sein, das Signalmittel in Abhängigkeit des Abstands der Assistenzsystems von der Arbeitsraumgrenze anzusteuern, beispielsweise mit einem Steuerstrom, der antiproportional zum Abstand ausgegeben wird.

Der Haltearm weist besonders bevorzugt zusätzlich zu der Assistenz-Bedieneinrichtung eine Arm-Bedieneinrichtung zum Verbringen des Haltearms in eine gewünschte Pose auf. Die Arm-Bedieneinrichtung ist vorzugsweise dazu eingerichtet, bei Kontakt zwischen einer Bedienperson und einem Armsegment das zugeordnete Gelenk freizugeben. Dies ist insbesondere bevorzugt wenn der Haltearms als passiver Haltearm mit aktiv gebremsten Gelenken ausgebildet ist. Es ist also vorzugsweise vorgesehen, dass bei Kontakt einer Bedienperson mit einem entsprechenden Armsegment nur das zugeordnete Gelenk freigegeben wird. Dadurch ist es möglich, intuitiv einzelne Gelenke zu bewegen und den Haltearm so segmentweise zu verstellen und in eine gewünschte Pose zu bringen. Dadurch ist eine genauere Positionierung möglich, da inkrementell jedes Segment separat verstellt werden kann. Es ist ebenso möglich, mehrere Armsegmente auf einmal zu kontaktieren, sodass mehrere Gelenke gleichzeitig freigegeben werden und so verstellbar sind. Dadurch ist es möglich, den Haltearm auf einfache Art und Weise, insbesondere intuitiv, in eine gewünschte Pose zu überführen. Dazu kann an dem Haltearm, insbesondere an den Segmenten, ein Kontaktmittel vorgesehen sein, welches mit der Arm-Bedieneinrichtung derart zusammenwirkt, dass die Arm-Bedieneinrichtung bei einem Kontakt zwischen dem Bediener und dem Kontaktmittel ein zugeordnetes Gelenk freigibt.

Gemäß einer weiteren bevorzugten Ausführungsform der Erfindung weisen die Gelenke Bremsen auf, mittels derer die Gelenke freigebbar und arretierbar sind. Die Bremsen dienen dazu, eine Bewegung der Armsegmente relativ zueinander, also eine Bewegung der Gelenke zu bremsen beziehungsweise zu verhindern. Sind die Bremsen gelöst, sind die Gelenke freigegeben.

Die Aufgabe wird ebenfalls gelöst durch ein System aus einem vorbeschriebenen Haltearm und einem chirurgischen mechatronischen Assistenzsystem.

Bezüglich des Verfahrens wird die Aufgabe gelöst durch ein Verfahren mit den Merkmalen des Anspruchs 7, insbesondere ein Verfahren zum Steuern eines an einen Haltearm für medizinische Zwecke gekoppelten chirurgischen mechatronischen Assistenzsystems mit den Schritten:
- Erfassen einer auf ein Steuern des Assistenzsystems gerichtete Bedienaktion eines Bedieners mittels einer an dem Haltearm angeordneten Bedieneinrichtung, und
- Bedarfsweises Übertragen eines die Bedienaktion repräsentierenden Anforderungssignals an die Assistenz-Schnittstelle zwecks Steuerung des chirurgischen mechatronischen Assistenzsystems.

Unter einem "bedarfsweisen" Übertragen des Anforderungssignals an die Assistenz-Schnittstelle soll vorliegend verstanden werden, dass das Übertragen des Anforderungssignals nicht zwingend auf jede Bedienaktion hin erfolgen muss. Vielmehr ist das Übertragen bevorzugt an Bedingungen geknüpft, wie beispielsweise eine weiter unten beschriebene Nichtverletzung einer Arbeitsraumbeschränkung durch das mechatronische Assistenzsystem.

Bevorzugt erfolgt ein Umwandeln des Anforderungssignals in ein Steuersignal für das chirurgische mechatronische Assistenzsystem erst im Assistenzsystem selbst. Alternativ erfolgt ein Umwandeln des Anforderungssignals in ein Steuersignal für das chirurgische mechatronische Assistenzsystem bereits in einer dem Haltearm zugeordneten Verarbeitungseinheit, die bevorzugt als Mikrocontroller bereitgestellt ist. Vorteilhafterweise kann somit Rechenbedarf im mechatronischen Assistenzsystem reduziert werden.

Das Verfahren weist den Schritt auf: Umwandeln der erfassten Bedienaktion in eine Datenstruktur, wobei die Datenstruktur bevorzugt einen Bewegungsvektor, insbesondere einen 3D-Bewegungsvektor und/oder einen Geschwindigkeitswert umfasst. Besonders bevorzugt weist die Datenstruktur nur einen 3D-Bewegungsvektor und einen Geschwindigkeitswert auf. Es erfolgt ein Erzeugen des Anforderungssignals aus der Datenstruktur.

Für das Verständnis der Erfindung wird darauf hingewiesen, dass im Rahmen des Verfahrens bevorzugt ein Auslesen von den Haltearm betreffenden Sensordaten und/oder Arbeitsraumdaten über die Übertragungseinrichtung erfolgt. Somit wird in vorteilhafter Weise die Steuerungsgenauigkeit und daraus resultierend auch die Sicherheit des zu steuernden chirurgische mechatronische Assistenzsystem erhöht.

Ferner wird zum Verständnis angegeben, dass das Verfahren vorzugsweise den Schritt aufweist: Umwandeln der erfassten Bedienaktion in eine Datenstruktur unter Berücksichtigung der den Haltearm betreffenden Sensordaten. Dazu wird bevorzugt der Bewegungsvektor, welcher unmittelbar d.h. ohne den Haltearm betreffende Transformation die Bedienaktion an der am Haltearm angeordneten Bedieneinrichtung repräsentiert, in einen resultierenden Bewegungsvektor transformiert.

Bevorzugt erfolgt eine Berechnung des resultierenden Bewegungsvektors derart, dass eine Transformationsmatrix berechnet wird, die die momentane Raum-Lage der Assistenz-Bedieneinrichtung, insbesondere eines Kontaktmittels, bezogen auf ein Basiskoordinatensystem des Haltearms repräsentiert und bevorzugt wenigstens eine rotatorische und eine translatorische Transformationskomponente aufweist. Bevorzugt wird weiter eine Übergangsmatrix berechnet, die die momentane Raum-Lage der Assistenz-Bedieneinrichtung, insbesondere des Kontaktmittels, bezogen auf einen Arbeitspunkt des mechatronischen Assistenzsystems repräsentiert. Weiter bevorzugt wird der Bewegungsvektor der Bedieneinrichtung von rechts an die Übergangsmatrix multipliziert, welche wiederum von rechts an die Transformationsmatrix multipliziert wird. Bevorzugt wird die Transformationsmatrix mit den Haltearm betreffenden Sensordaten und mechanischen/konstruktiven Parametern des Haltearms besetzt. Weiter bevorzugt wird die Übergangsmatrix mit Sensordaten betreffend das mechatronische Assistenz-system und mechanischen/konstruktiven Parametern betreffend das mechatronischen Assistenzsystems besetzt.

Zum Verständnis der Erfindung wird darauf hingewiesen, dass in einer weiteren bevorzugten Ausgestaltung das Verfahren den Schritt aufweist: Ermitteln, ob das chirurgische mechatronische Assistenzsystem bei Ansteuerung gemäß der Anforderungssignal innerhalb eines zulässigen Arbeitsraums verbleiben würde. Bevorzugt ist der zulässige Arbeitsraum des chirurgischen mechatronische Assistenzsystems in einem intraoperativen Navigationssystem und/oder einer Planungsdatei hinterlegt. Soll beispielsweise ein von einem mechatronischen Assistenzsystem umfasstes Linearmodul (z.B. zum Einfahren/Ausfahren eines Endoskops) entlang einer festgelegten Translationsachse gesteuert werden, so ist der zulässige Arbeitsraum bevorzugt durch die maximal zulässige Einfahrtiefe des Endoskops in den Patienten definiert.

Dem Verständnis dient ferner, dass ein Übertragen des Anforderungssignals an das chirurgische mechatronische Assistenzsystem bevorzugt erfolgt, wenn das chirurgische mechatronische Assistenzsystem bei Ansteuerung gemäß der Anforderungssignal innerhalb eines zulässigen Arbeitsraums verbleiben würde. In diesem Fall wird ein Anforderungssignal nur Übertragen, falls keine Arbeitsraumverletzung durch das Assistenzsystem gegeben wäre.

Zum weiteren Verständnis wird angegeben, dass das Verfahren alternativ oder zusätzlich den Schritt aufweisen kann: Filtern eines Anforderungssignals, wenn das chirurgische mechatronische Assistenzsystem bei Ansteuerung gemäß der Anforderungssignal den zulässigen Arbeitsraum verlassen würde. Das Verfahren kann ein Filtern des Anforderungssignals insoweit vorsehen, als dass das chirurgische mechatronische Assistenzsystem bei Ansteuerung gemäß der Anforderungssignal einen zulässigen Arbeitsraum verlassen würde und/oder sich der Grenze des zulässigen Arbeitsraums bis auf einen vorgegebenen Abstand nähern würde. Bevorzugt erfolgt ein Filtern "insoweit" durch Skalieren des Anforderungssignals. Ein Skalieren des Anforderungssignals erfolgt bevorzugt hinsichtlich einer Geschwindigkeit mittels derer sich beispielsweise ein von einem mechatronischen Assistenzsystem umfasstes Linearmodul bewegen soll. Diese Filterung würde sich für einen (beispielhaften) Endoskop- Bediener derart manifestieren, dass sich das Endoskop mit durch den Bediener an der Bedieneinheit durch Gestensteuerung vorgegebenen Geschwindigkeit der Grenze des zulässigen Arbeitsraums nähert um sich dort automatisch zu verlangsamen oder um zu stoppen.

Ebenfalls für das Verständnis der Erfindung dient Folgendes: Besonders bevorzugt erfolgt ein Ausgeben eines durch den Bediener wahrnehmbaren Warnsignals über die Bedieneinrichtung wenn für das chirurgische mechatronische Assistenzsystem eine Arbeitsraumbeschränkung gegeben ist. Bevorzugt erfolgt das Ausgeben des Warnsignals zusammen mit der eben beschrieben Annäherung an die Grenze des zulässigen Arbeitsraums.

Das Verfahren kann den Schritt aufweisen: Bestimmen, ob eine über die Bedieneinrichtung erfasste Bedienaktion auf die Freigabe eines Gelenks oder eine Bewegung des mechatronischen Assistenzsystems gerichtet ist. Dies ist insbesondere vorteilhaft wenn z.B. Assistenz-Bedieneinrichtung und Arm-Bedieneinrichtung über ein- und dasselbe Kontaktmittel bedient werden. Das Bestimmen erfolgt bevorzugt durch Auswerten einer Spracheingabe eines Nutzers, beispielsweise durch die Ansprache "Freigabemodus Gelenke" oder "Steuermodus Assistenzsystem".

Besonders bevorzugt, ist die zu erfassende Bedienaktion des Bedieners eine Geste, insbesondere eine Fingergeste.

Es soll verstanden werden, dass der erfindungsgemäße Haltearm und das erfindungsgemäße Verfahren gleiche und ähnliche Aspekte haben, wie sie insbesondere in den Unteransprüchen niedergelegt sind. Daher wird für die bevorzugten Ausführungsformen des Verfahrens sowie deren Vorteile auch auf die obige Beschreibung zum Haltearm vollumfänglich Bezug genommen.

Nachstehend wird die Erfindung anhand von drei Ausführungsbeispielen unter Bezugnahme auf die beiliegenden Figuren näher erläutert. Dabei zeigen:
- Figur 1: ein ersten Ausführungsbeispiel eines Haltearms;
- Figur 2: schematisch die Funktionsweise des erfindungsgemäßen Haltearms;
- Figur 3: eine Seitenansicht eines erfindungsgemäßen Haltearms, bei dem ein Steuerungsring der Assistenz-Bedieneinrichtung zu sehen ist;
- Figur 4: eine Seitenansicht eines Haltearms gemäß einem dritten Ausführungsbeispiel, bei dem Kontaktmittel der Assistenz-Bedieneinrichtung zu sehen sind;
- Figur 5: eine teilweise aufgebrochene Ansicht des Haltearms aus Figur 4;
- Figur 6: den Haltearm aus Figur 4, mit einer externen Steuereinheit und einem Assistenzsystem gekoppelt;
- Figur 7: eine schematische Darstellung eines Armsegments zur Verdeutlichung der Übertragungseinrichtung; und
- Figur 8: ein Flussdiagramm eines erfindungsgemäßen Verfahrens.

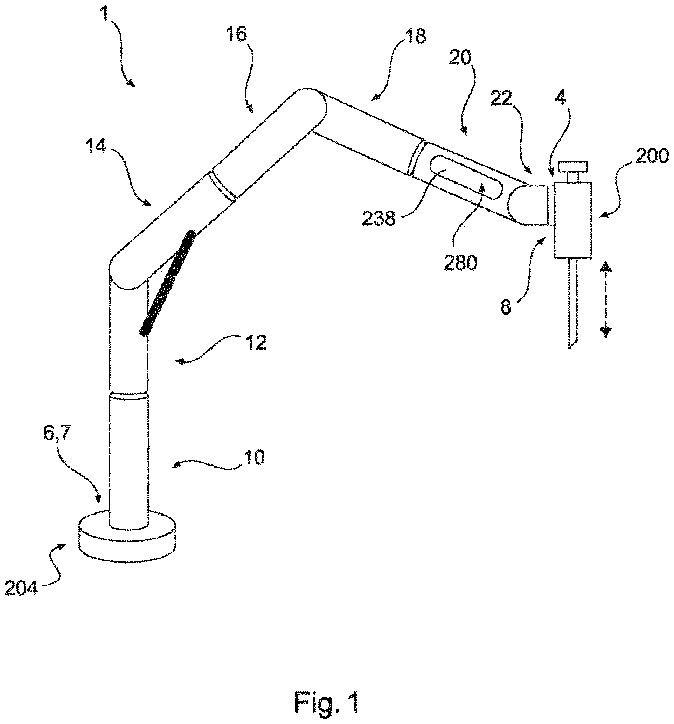

Figur 1 zeigt einen Haltearm 1 für medizinische Zwecke, der vorliegend zum Halten eines chirurgischen mechatronischen Assistenzsystems 200, das in Form eines mechatronischen Endoskops bereitgestellt ist.

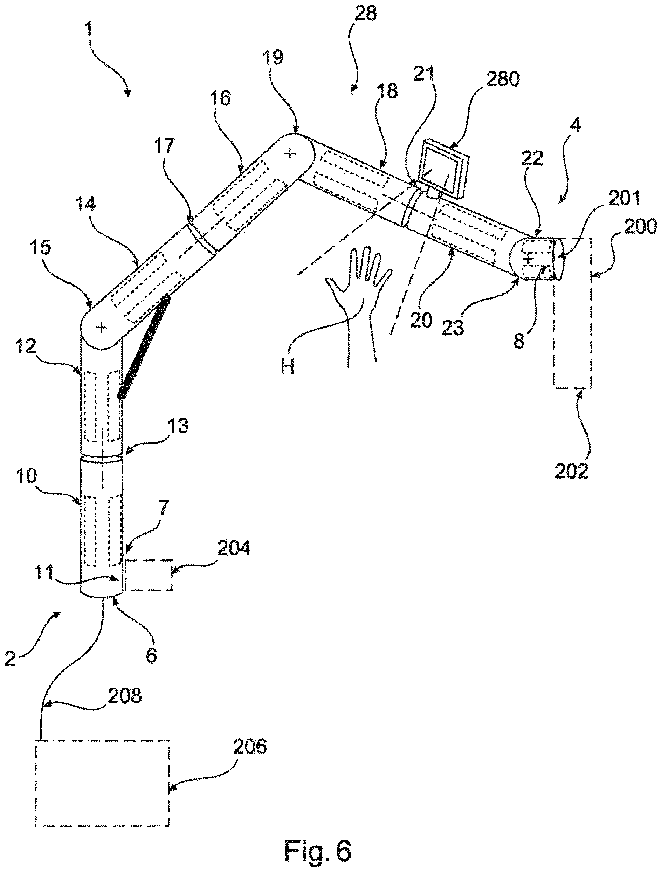

Der Haltearm 1 weist ein proximales Ende 2 und ein distales Ende 4 auf. An dem proximalen Ende 2 sind eine Basis-Schnittstelle 6 und eine mechanische Schnittstelle 7 ausgebildet. Die mechanische Schnittstelle 7 dient dazu, den Haltearm 1 an einer Basis 204, beispielsweise an einem Operationstisch, zu befestigen. Die Basis-Schnittstelle 6 dient zur Übergabe von Energie sowie zur Kopplung des Haltearms 1 mit einer externen Steuereinheit 206 (vgl. Fig. 6). An dem distalen Ende 4 ist eine Assistenz-Schnittstelle 8 vorgesehen, über die das mechatronische Assistenzsystem 200 an den Haltearm 1 gekoppelt und gesteuert werden kann. Der Haltearm 1 gemäß Figur 1 weist sieben Armsegmente 10, 12, 14, 16, 18, 20, 22 auf, die jeweils im Wesentlichen stabförmig sind und bis auf das letzte Armsegment 22 alle im Wesentlichen eine gleiche Länge aufweisen.

Der Halterarm 1 weist eine Assistenz-Bedieneinrichtung 280 auf, die am vorletzten Armsegment 20 angeordnet und dazu eingerichtet ist, eine auf ein Steuern des Assistenzsystems 200 gerichtete Bedienaktion eines Bedieners zu erfassen und bedarfsweise ein Übertragen eines die Bedienaktion repräsentierenden Anforderungssignals an die Assistenz-Schnittstelle 8 zwecks Steuerung des chirurgischen mechatronischen Assistenzsystems 200 zu bewirken.

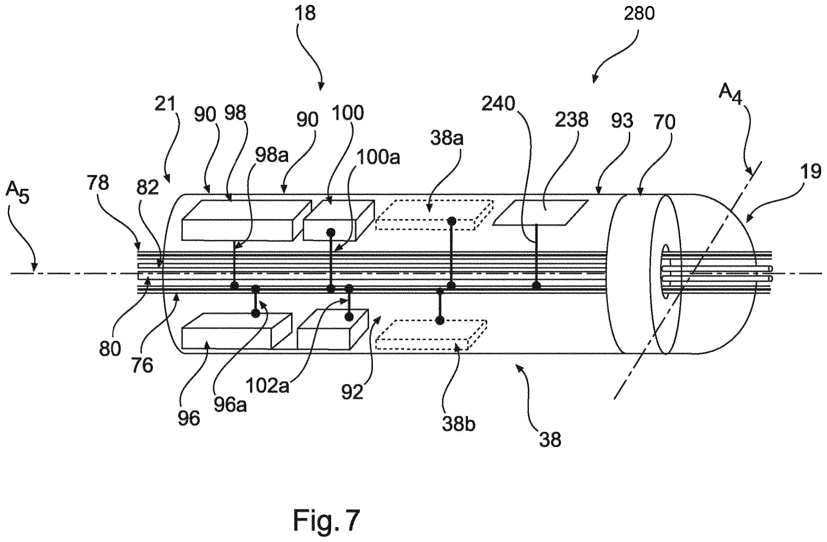

Figur 1 zeigt schematisch einen nur in Teilen dargestellten Haltearm 1 mit angeschlossenem mechatronischen Assistenzsystem 200, wobei das Assistenzsystem 200 über eine Assistenz-Schnittstelle 8 des Haltearms 1 an ein Bussystem 76 des Haltearms 1 angekoppelt ist. Der Haltearm 1 weist vorliegend eine Assistenz-Bedieneinrichtung 280 mit einem als berührungsempfindliches Sensorfeld ausgebildeten Kontaktmittel 238 auf, wobei das berührungsempfindliche Sensorfeld eine Alternative zu dem erfindungsgemäßen Steuerungsring darstellt. Das als berührungsempfindliches Sensorfeld ausgebildete Kontaktmittel 238 der Assistenz-Bedieneinrichtung 280 liefert Bewegungskoordinaten x1, y1; x2, y2; xn, yn auf einer berührungsempfindlichen Oberfläche (nicht gezeigt) und den jeweiligen Erfassungszeitpunkt t1; t2; tn an eine serielle Schnittstelle 240 (vgl. Fig. 7)

Die eine Assistenz-Bedieneinrichtung 280 weist eine zugeordnete Verarbeitungseinheit 100 auf, die im vorliegenden Beispiel einen ersten Mikrokontroller 110 und einen zweiten Mikrokontroller 120 aufweist. Die beiden Mikrokontroller 110, 120 müssen nicht notwendigerweise als diskrete Bausteine vorliegen, sondern können vielmehr auch durch ein und denselben Baustein realisiert sein. Im vorliegenden Beispiel sind die beiden Mikrokontroller 110, 120 als diskrete Bausteine bereitgestellt, die über eine Datenverbindung 115 kommunikativ gekoppelt sind.

Der erste Mikrokontroller 110 der Verarbeitungseinheit 100 des Haltearms 1 empfängt die Bewegungskoordinaten x1, y1; x2, y2; xn, yn und wandelt zwei aufeinanderfolgende Koordinaten x1, y1; x2, y2 in eine interne Datenstruktur (dreidimensionalen Bewegungsvektor) um. Damit ist die Bewegungsrichtung r des Bewegungspfades P, der auf dem als berührungsempfindliches Sensorfeld ausgebildete Kontaktmittel 238 durch den Bediener abgefahren wird, festgelegt. Falls nur mit einem Finger über das als berührungsempfindliches Sensorfeld ausgebildete Kontaktmittel 238 gefahren wird, handelt es sich um eine zweidimensionale Bewegung, die dritte Koordinate im Bewegungsvektor r ist entsprechend 0.

Aus den beiden Erfassungszeiten t1; t2 berechnet der erste Mikrokontroller 110 eine Bewegungsgeschwindigkeit v. Diese wird zusammen mit dem Bewegungsvektor r an den zweiten Mikrokontroller 120 übertragen. Im Falle, dass ein Bediener zwei Finger gleichzeitig auf dem als berührungsempfindliches Sensorfeld ausgebildeten Kontaktmittel 238 bewegt, werden jeweils gleichzeitig zwei Koordinaten und Erfassungszeiten übertragen. Bei drei Fingern entsprechende drei.

Soll beispielsweise ein von einem mechatronischen Assistenzsystem 200 umfasstes Linearmodul entlang einer festgelegten Translationsachse gesteuert werden (vgl. Fig. 1, gestrichelte Linie) so ist der erste Mikrocontroller 110 bevorzugt dazu ausgebildet, einen Bewegungsvektor r = (0, 0, 1) zu erzeugen, wenn der Bediener auf dem als berührungsempfindliches Sensorfeld ausgebildeten Kontaktmittel 238 beispielsweise eine Zweifinger-Geste ausführt, wobei die zwei Finger bis zur Berührung aufeinander zu bewegt werden. Der Bewegungsvektor r = (0, 0, 1), umgewandelt in ein Anforderungssignal m, würde, sofern bedarfsweise übertragen an das mechatronischen Assistenzsystem 200, ein Einfahren des Linearmoduls bewirken. Dementsprechend ist der erste Mikrocontroller 110 bevorzugt weiter dazu ausgebildet einen Bewegungsvektor r = (0, 0, -1) zu erzeugen, wenn die zwei Finger, beginnend mit Ihrer Berührung, voneinander weg bewegt werden. Der Bewegungsvektor r = (0, 0, -1), umgewandelt in ein Anforderungssignal m, würde, sofern bedarfsweise übertragen an das mechatronischen Assistenzsystem 200, ein Ausfahren des Linearmoduls bewirken.

Der erste Mikrokontroller 110 der Verarbeitungseinheit 100 ist kommunikativ mit einem internen Bussystem 76 gekoppelt, das Teil einer Übertragungseinrichtung ist, die innerhalb des Haltearms 1 angeordnet ist und die Assistenz-Schnittstelle 8 mit einer Basis-Schnittstelle (nicht gezeigt) des Haltearms 1 zum Übertragen Signalen zwischen den Schnittstellen verbindet. Der erste Mikrokontroller 110 der Verarbeitungseinheit 100 ist vorliegend dazu ausgebildet, den Haltearm 1 betreffende Sensordaten (SI1, SI2, SIN) über das Bussystem 76 auszulesen. Aus diesen Sensordaten (SI1, SI2, SIN) berechnet der erste Mikrokontroller 110 der Verarbeitungseinheit 100 die Lage des Haltearms im Raum, um die Lage des Kontaktmittels 238 zu bestimmen und diese in die Berechnung des (resultierenden) Bewegungsvektors r einzubeziehen.

Bevorzugt erfolgt eine Berechnung des resultierenden Bewegungsvektors derart, dass eine Transformationsmatrix berechnet wird, die die momentane Lage Kontaktmittels 238 im Raum bezogen auf ein Basiskoordinatensystem des Haltearms 1 repräsentiert und bevorzugt wenigstens eine rotatorische und eine translatorische Transformationskomponente aufweist; und/oder dass eine Übergangsmatrix berechnet wird, die die momentane Lage des Kontaktmittels 238 im Raum bezogen auf einen Arbeitspunkt des mechatronischen Assistenzsystems 200 repräsentiert; und/oder dass der durch den ersten Mikrocontroller 110 ermittelte Bewegungsvektor r des Kontaktmittels von rechts an die Übergangsmatrix multipliziert wird, welche wiederum von rechts an die Transformationsmatrix multipliziert wird.

Bewegungsvektor r und Bewegungsgeschwindigkeit v bilden eine Datenstruktur, die über die Datenverbindung 115 vom erste Mikrokontroller 110 an den zweiten Mikrokontroller 120 übertragen wird. Der zweite Mikrokontroller 120 der Verarbeitungseinheit 100 ist ebenfalls kommunikativ mit dem internen Bussystem 76 gekoppelt und ist ausgebildet an dem internen Bussystem 76 abzufragen, ob Arbeitsraumbeschränkungen (CI,C2,..Cn) in einer Planungsdatei oder beispielsweise in einem intraoperativen Navigationssystem vorliegen. Des Weiteren ist der zweiten Mikrokontroller 120 dazu eingerichtet bedarfsweise ein Anforderungssignal m an die Assistenz-Schnittstelle 8 zwecks Steuerung des chirurgischen mechatronischen Assistenzsystems 200 zu übertragen. Ein Bedarf ist beispielweise gegeben, falls der zweiten Mikrokontroller 120 der Verarbeitungseinheit 100 ermittelt, dass das chirurgische mechatronische Assistenzsystem 200 bei Ansteuerung gemäß dem Anforderungssignal m innerhalb eines zulässigen Arbeitsraums verbleiben würde. In diesem Fall wird also das die Bedienaktion repräsentierende Anforderungssignals m an die Assistenz-Schnittstelle 8 weitergeleitet und von dem chirurgischen mechatronische Assistenzsystem 200 in ein Steuersignal zur Ansteuerung interner Motoren umgewandelt.

Falls der zweiten Mikrokontroller 120 der Verarbeitungseinheit 100 ermittelt, dass das chirurgische mechatronische Assistenzsystem 200 bei einer Ansteuerung gemäß dem Anforderungssignals m den zulässigen Arbeitsraum verlassen würde (z.B. "Endoskop berührt das Zielgebiet in unerwünschter Weise"), so gibt der zweite Mikrokontroller 120 der Assistenz-Bedieneinrichtung 280 eine Warnung w aus, die im Signalmittel 239 der Verarbeitungseinheit 100 in ein für den Bediener wahrnehmbares Warnsignal, vorliegend beispielhaft einen Warnton W', umgewandelt wird. In diesem Fall ist kein Bedarf einer Übertragung des Anforderungssignal m an die Assistenz-Schnittstelle 8 gegeben, da dies unter Umständen zur Verletzung des Patienten führen könnte.

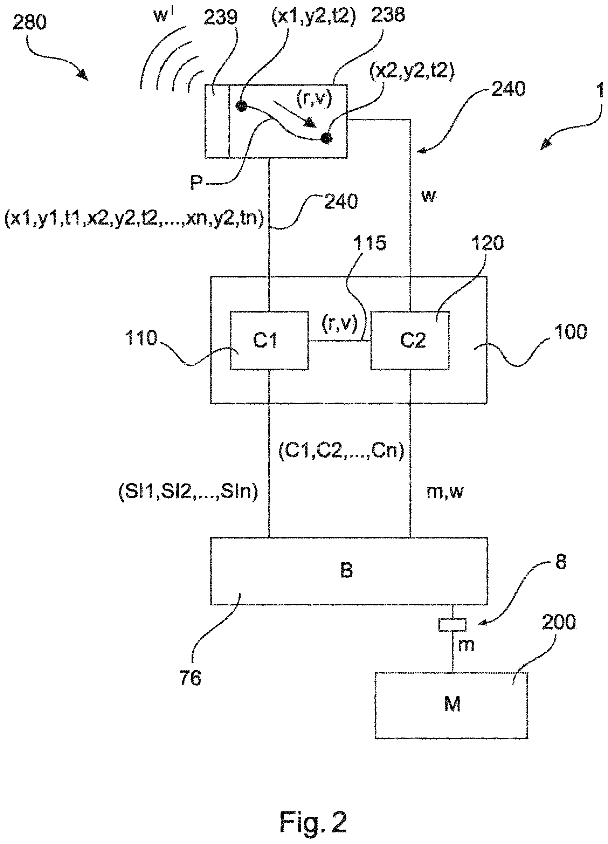

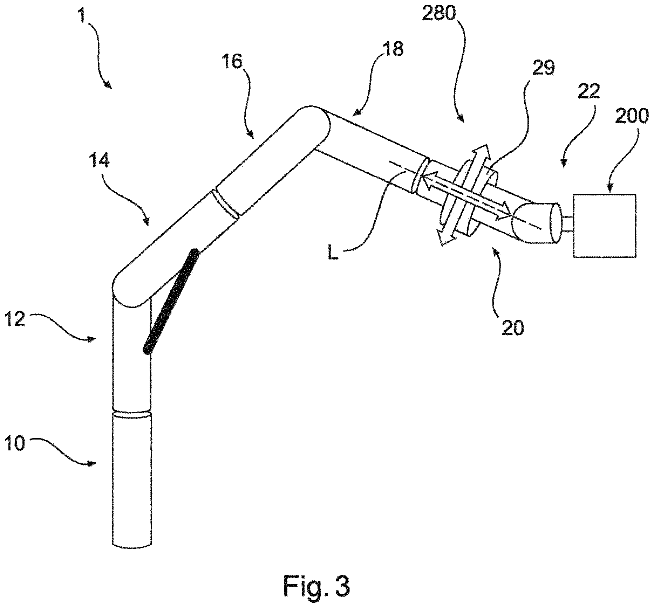

Figur 3 zeigt einen Haltearm 1 gemäß einem zweiten Ausführungsbeispiel der Erfindung. Der Haltearm 1 ist im Wesentlichen identisch zu dem aus den Figuren 1 und 2 gebildet und weist sieben Armsegmente 10, 12, 14, 16, 18, 20, 22 auf. Im Unterschied zu den vorherigen Ausführungsbeispielen weist die Arm-Bedieneinrichtung 280 des Haltearms 1 einen Steuerungsring 29 auf. Dieser ist vorliegend um das vorletzte Armsegment 20 herum angeordnet und - wie durch die Pfeile angedeutet - wenigstens umfänglich um das Armsegment 20 um die Längsachse L des Armsegments 20 drehbar bzw. axial entlang der Längsachse L beweglich.

Rotiert der Bediener den Steuerungsring 29 um die Längsachse L, so wird dies durch die Verarbeitungseinheit 100 (vgl. Fig. 2) erkannt und ein entsprechendes Anforderungssignal, dass die Rotationsrichtung, den Rotationswinkel, die Rotationsgeschwindigkeit und/oder die Rotationsbeschleunigung aufweisen bzw. repräsentieren kann über das interne Bussystem 76 (vgl. Fig. 2) an das chirurgische mechatronische Assistenzsystems 200 gesendet. Die Steuerungssoftware des Assistenzsystems 200 empfängt das Anforderungssignal und berechnet daraus einen oder mehrere Steuerungsbefehle und sendet diese an die Motoren (nicht gezeigt) des Assistenzsystems 200. Bewegt der Bediener den Steuerungsring 29 entlang der Längsachse L vor oder zurück, erkennt dies die Verarbeitungseinheit 100 und sendet die Bewegungsrichtung sowie die Bewegungsstrecke und die Bewegungsgeschwindigkeit sowie die Bewegungsbeschleunigung über das interne Bussystem 76 an das Assistenzsystem 200.

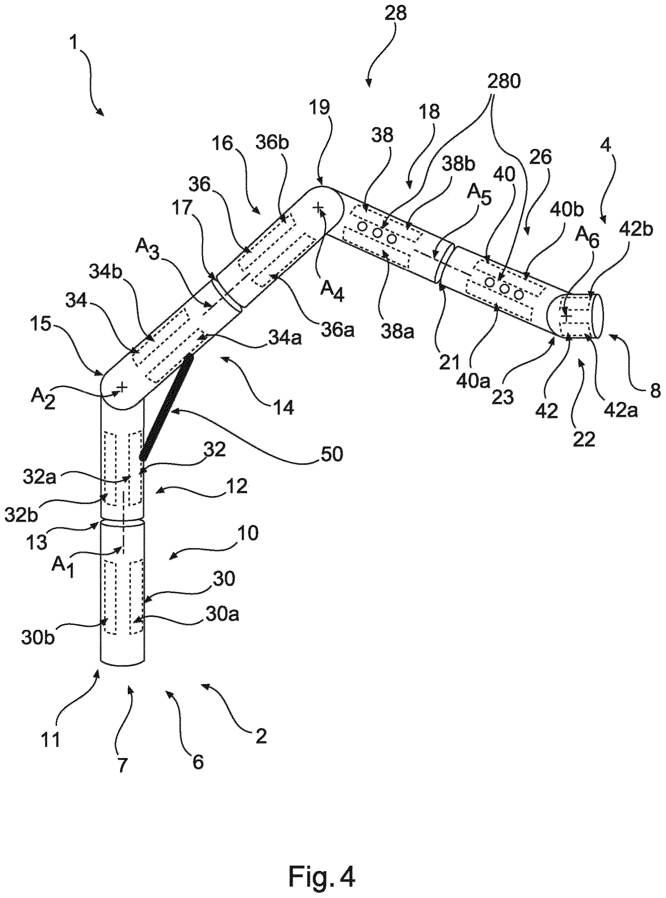

Figur 4 zeigt einen Haltearm 1 für medizinische Zwecke, insbesondere zum Halten eines chirurgischen mechatronischen Assistenzsystems und/oder chirurgischen Instruments (nicht gezeigt) gemäß einem dritten Ausführungsbeispiel. Der Haltearm 1 weist eine erfindungsgemäße Assistenz-Bedieneinrichtung 280 auf, die vorliegend als Gruppe von Knöpfen bereitgestellt ist. Zusätzlich zu der Assistenz-Bedieneinrichtung 280 ist eine Arm-Bedieneinrichtung 28 vorgesehen, mittels derer wenigstens ein Gelenk, bevorzugt jedes Gelenk 11, 13, 15, 17, 19, 21, 23 freigebbar und arretierbar ist, so dass der Haltearm 1 in eine gewünschte Pose bringbar ist. Die Arm-Bedieneinrichtung 28 ist dazu eingerichtet bei Kontakt zwischen einer Bedienperson und einen der zwei oder mehr Armsegmente (10, 12, 14, 16, 18, 20, 22) das zugeordnete Gelenk (11, 13, 15, 17, 19, 21, 23) freizugeben. Dies wird im Folgenden genauer erläutert.

Der Haltearm 1 weist ein proximales Ende 2 und ein distales Ende 4 auf. An dem proximalen Ende 2 sind eine Basis-Schnittstelle 6 und eine mechanische Schnittstelle 7 ausgebildet Die mechanische Schnittstelle 7 dient dazu, den Haltearm 1 an einer Basis, wie insbesondere an einem Operationstisch, zu befestigen. Die Schnittstelle 7 dient zur Übergabe von Energie sowie zur Kopplung des Haltearms 1 mit einer externen Steuereinheit (vgl. Fig. 6). An dem distalen Ende 4 ist eine zweite Schnittstelle 8 vorgesehen, über die ein mechatronisches Assistenz-system und/oder ein chirurgisches Instrument, wie insbesondere ein Manipulator, an den Haltearm 1 koppelbar ist. Vorzugsweise wird hier ein Manipulator zum Halten und Manipulieren eines Endoskops angeordnet.

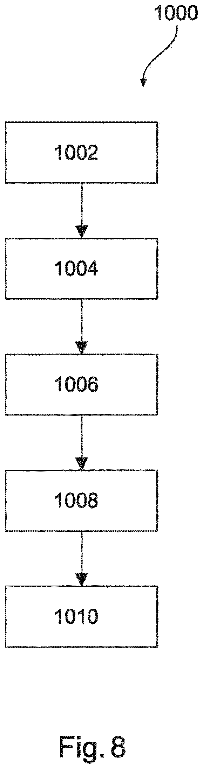

Der Haltearm 1 gemäß Figur 4 weist sieben Armsegmente 10, 12, 14, 16, 18, 20, 22 auf, die jeweils im Wesentlichen stabförmig sind und bis auf das letzte Armsegment 22 alle im Wesentlichen eine gleiche Länge aufweisen. Die sieben Armsegmente 10, 12, 14, 16, 18, 20, 22 sind jeweils mit Gelenken 11, 13, 15, 17, 19, 21, 23 miteinander gekoppelt, wobei das nullte Gelenk 11 den Haltearm 1 mit der Basis (in Fig. 4 nicht gezeigt, s. Fig. 1) koppelt. Die Gelenke 13, 15, 17, 19, 21, 23 sind gemäß diesem Ausführungsbeispiel sämtlich als rotatorische Gelenke mit jeweils einem Freiheitsgrad ausgebildet. Gemäß diesem Ausführungsbeispiel ist dem nullten Segment 10 das nullte Gelenk 11 zugeordnet, dem ersten Armsegment 12 das erste Gelenk 13 zugeordnet, dem zweiten Armsegment 14 das zweite Gelenk 15 zugeordnet, dem dritten Armsegment 16 das dritte Gelenk 17 zugeordnet, dem vierten Armgelenk 18 das vierte Gelenk 19 zugeordnet, dem fünften Armsegment 20 das fünfte Gelenk 21 zugeordnet, und dem sechsten Armsegment 22 ist das sechste Gelenk 23 zugeordnet. Das Gelenk 11 ist als translatorisches Gelenk ausgebildet, sodass das Armsegment 10 teleskopartig verlängerbar ist, um so den Haltearm 1 in der Höhe zu verstellen, wie später mit Bezug auf Figur 8 erläutert werden wird. Die Gelenke 13, 15, 17, 19, 21, 23 weisen jeweils Schwenkachsen A₁, A₂, A₃, A₄, A₅, A₆ auf, wobei jeweils benachbarte Gelenke Schwenkachsen haben, die senkrecht zueinander sind. Hierdurch wird eine einfache Positionierung des distalen Endes 4 im Raum erreicht.

Der Haltearm 1 gemäß Figur 4 weist ferner eine Arm-Bedieneinrichtung 28 auf. Mittels der Arm-Bedieneinrichtung 28 ist der Haltearm 1 in eine gewünschte Pose verbringbar, wobei die Arm-Bedieneinrichtung 28 dazu eingerichtet ist, bei Kontakt zwischen einer Bedienperson und einem der sieben Armsegmente das zugeordnete Gelenk freizugeben. Dazu weist die Arm-Bedieneinrichtung 28 gemäß diesem Ausführungsbeispiel sieben Kontaktabschnitte 30, 32, 34, 36, 38, 40, 42 auf, wobei an jedem Armsegment 10, 12, 14, 16, 18, 20, 22 jeweils ein Kontaktmittel 30, 32, 34, 36, 38, 40, 42 angeordnet ist. So ist am nullten Armsegment 10 ein nulltes Kontaktmittel 30 angeordnet, am ersten Armsegment 12 ein erstes Kontaktmittel 32, am zweiten Armsegment 14 ein zweites Kontaktmittel 34, am dritten Armsegment 16 ein drittes Kontaktmittel 36, am vierten Armsegment 18 ein viertes Kontaktmittel 38, am fünften Armsegment 20 ein fünftes Kontaktmittel 40 und am sechsten Armsegment 22 ein sechstes Kontaktmittel 42 angeordnet.

Ferner ist gemäß diesem Ausführungsbeispiel vorgesehen, dass jedes Kontaktmittel 30, 32, 34, 36, 38, 40, 42 jeweils zwei im Wesentlichen gegenüberliegend angeordnete Kontaktmittelelemente 30a, 30b, 32a, 32b, 34a, 34b, 36a, 36b, 38a, 38b, 40a, 40b, 42a, 42b aufweist. Die Kontaktmittel 30, 32, 34, 36, 38, 40, 42 dienen dazu, einen Kontakt eines Bedieners mit dem entsprechenden Armsegment 10, 12, 14, 16, 18, 20, 22 zu erfassen. Beim Ergreifen eines Armsegments 10, 12, 14, 16, 18, 20, 22 kommt der Bediener in Kontakt mit beiden Kontaktmittelelementen 30a, 30b bis 42a, 42b, und nur bei Kontakt mit beiden Kontaktmittelelementen 30a, 30b bis 42a, 42b eines Kontaktmittels 30 bis 42 wird das zugeordnete Gelenk freigegeben. Das heißt, beim Ergreifen des ersten Armsegments 12 und gleichzeitigem Inkontaktkommen mit den beiden Kontaktmittelelementen 32a, 32b wird durch die Arm-Bedieneinrichtung 28 das erste Gelenk 13 freigegeben. Dadurch ist es möglich, dass der Bediener den Haltearm 1 beziehungsweise die Armsegmente 12 bis 22 um die Achse A₁ verschwenken kann. Bei Loslassen eines der beiden oder beider Kontaktmittelelemente 32a, 32b wird das Gelenk 13 wieder arretiert, und ein Verschwenken um die Achse A₁ ist nicht mehr möglich. Auch bei einer unbeabsichtigten Berührung nur eines der beiden Kontaktmittelelemente 32a, 32b, beispielsweise mit einem Arm oder Ellenbogen des Bedieners, wird das Gelenk 13 nicht freigegeben, und der Haltearm 1 bleibt im arretierten Zustand und hält seine Pose.

Entsprechendes gilt für das zweite Armsegment 14. Auch hier weist das zweite Kontaktmittel 34 zwei Kontaktmittelelemente 34a, 34b auf, die im Wesentlichen gegenüberliegend umfänglich an dem Armsegment 14 vorgesehen sind. Bei Ergreifen dieses Armsegments 14 und Inkontaktkommen mit den beiden Kontaktmittelelementen 34a, 34b wird dieser Kontakt durch die Arm-Bedieneinrichtung 28 erfasst, und das dem Armsegment 14 zugeordnete Gelenk 15 freigegeben. Nun ist ein Verschwenken um die Achse A₂ möglich, sodass das distale Ende 4, bezogen auf Figur 4, nach oben beziehungsweise unten verschwenkt werden kann. Gleichzeitig bleiben alle weiteren Gelenke 13, 17, 19, 21, 23 arretiert, sodass in diesen keine Bewegung stattfindet.

Die Arm-Bedieneinrichtung 28 kann dazu einen Kontroller oder Mikroprozessor aufweisen, der dazu eingerichtet ist, einen Kontakt zwischen Kontaktmittelelementen 30a, 30b bis 42a, 42b zu erfassen und in elektrische Signale zu übertragen.

Die Kontaktmittel 30 beziehungsweise die Kontaktmittelemente 30a, 30b bis 42a, 42b sind gemäß diesem Ausführungsbeispiel und alternativ zu der beanspruchten Erfindung als berührungsempfindliche Sensoren ausgebildet und erfassen einen Druck eines Kontakts zwischen dem Bediener und dem entsprechenden Kontaktmittelelement 30a, 30b bis 42a, 42b. Vorzugsweise sind die Kontaktmittelelemente 30a, 30b bis 42a, 42b als kapazitive berührungsempfindliche Sensoren ausgebildet.

Bei dem dargestellten Haltearm 1 ist es auch möglich, dass ein Bediener zwei Armsegmente, beispielsweise das Armsegment 14 und das Armsegment 18, gleichzeitig ergreift und so gleichzeitig die Kontaktmittelelemente 34a, 34b und 38a, 38b kontaktiert. Folglich werden die Gelenke 15 und 19 freigegeben, und ein Verschwenken sowohl um die Achse A₂ als auch um die Achse A₄ ist möglich. Bei dieser gleichzeitigen Freigabe ist es möglich, eine Winkelorientierung der Armsegmente 18 und 20 im Raum beizubehalten, während nur die Armsegmente 34, 36 verschwenkt werden. So ist auch eine translatorische Bewegung des distalen Endes 4 möglich. In einer bevorzugten Ausgestaltung des Haltearms werden bei dem gleichzeitigen Kontaktieren von zwei Armsegmenten, gemäß diesem Beispiel der Armsegmente 14 und 18 nicht die Gelenke 15 und 19 freigegeben, sondern alle zwischen diesen Armsegmenten 14 und 18 liegenden Gelenke, also gemäß diesem Ausführungsbeispiel die Gelenke 17 und 19. Das Gelenk 15 bleibt arretiert. Der Haltearm 1 kann nun so in seiner Pose verändert werden, dass eine Rotation um die Achse A3 und die Achse A4 möglich ist. Dies ist eine besonders intuitive Bedienung des Haltearms. Entsprechend werden, beispielsweise bei dem Kontakt zwischen Bediener und den Haltearmsegmenten 12 und 20, die Gelenke 15, 17, 19 und 21 freigegeben.

Weiterhin ist in der Figur 4 zu erkennen, dass der Haltearm 1 über eine Gewichtskompensationseinrichtung 50 verfügt. Die Gewichtskompensationseinrichtung 50 weist gemäß diesem Ausführungsbeispiel ein Gasdruckfederelement auf, welches mit dem Armsegment 14 und dem Armsegment 12 gekoppelt ist. Alternativ kann die Gewichtskompensationseinrichtung auch einen Seilzug und/oder ein ausbalanciertes Gegengewicht aufweisen. Bei dem Haltearm 1 gemäß Figur 4 lastet auf dem Gelenk 15 um seine Rotationsachse A2 das größte Moment. Folglich ist es bevorzugt, genau dieses Gelenk 15 mittels der Gewichtskompensationseinrichtung 50 abzustützen. Bei Freigabe des Gelenks 15, durch Kontaktieren des Armsegments 14, wird also ein Gewicht, welches auf dem Armsegment 14 lastet, aufgrund der weiteren Armsegmente 16, 18, 20, 22 und eines an der Schnittstelle 8 angeordneten Manipulators durch die Gewichtskompensationseinrichtung 50 abgestützt, sodass das distale Ende 4 bei Ergreifen des Segments 14 nicht sofort "absackt".

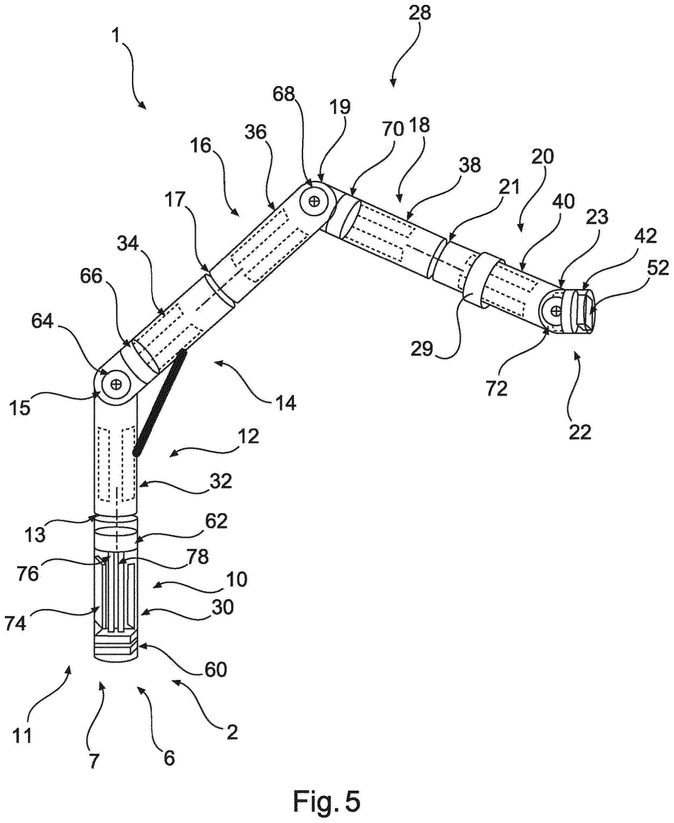

Figur 5 bei dem Haltearm 1 zusätzlich zu den bereits in Figur 4 gezeigten Elementen die Bremsen 60, 62, 64, 66, 68, 70, 72 dargestellt, mittels derer die Gelenke 11, 13, 15, 17, 19, 21, 23 freigebbar und arretierbar sind. Gleiche und ähnliche Elemente sind mit gleichen Bezugszeichen wie in Figur 4 versehen, und insofern wird vollumfänglich auf die obige Beschreibung Bezug genommen. Auch wenn in Figur 5 die Bezugszeichen an den Kontaktmittelelementen der Kontaktmittel 30, 32, 34, 36, 38, 40, 42 aus Klarheitsgründen nicht gezeigt sind, sind diese gleichwohl vorhanden, wie sich aus einem Vergleich der Figuren 4 und 5 ergibt. Im Gegensatz zum Haltearm 1 der Figur 4 weist der Haltearm 1 der Figur 5 eine Arm-Bedieneinrichtung 280 mit einem Steuerungsring 29 auf.

Jedem Gelenk 11, 13, 15, 17, 19, 21, 23 ist jeweils eine Bremse 60, 62, 64, 66, 68, 70, 72 zugeordnet. Dem Gelenk 11 ist die Bremse 60 zugeordnet, dem Gelenk 13 die Bremse 62, dem Gelenk 15 die Bremse 64, dem Gelenk 17 die Bremse 66, dem Gelenk 19 die Bremse 68, dem Gelenk 21 die Bremse 70 und dem Gelenk 23 die Bremse 72. Alle Bremsen 60 bis 72 sind als elektromagnetische Bremsen mit einem Permanentmagnet ausgebildet, derart, dass diese unbestromt in einen arretierten Zustand vorgespannt sind. Der Permanentmagnet ist derart ausgelegt, dass dieser das jeweilige Gelenk alleine arretieren kann und die Pose des Haltearms 1 gehalten wird. In dem nullten Armsegment 10 ist eine elektronische Steuereinheit 74 vorgesehen. Diese ist über ein Bussystem 76 (in Figur 5 nur im Armsegment 10 gezeigt; vgl. Fig. 7) mit allen Kontaktmitteln 30 bis 42 der Arm-Bedieneinrichtung 28 sowie mit allen Bremsen 60 bis 72 gekoppelt. Zur Energieversorgung der Bremsen 60 bis 72 sowie der Kontaktmittel 30 bis 42 ist ferner eine Energieleitung 78 vorgesehen, die über die Schnittstelle 6 am proximalen Ende 2 des Haltearms 1 mit einer Energiequelle koppelbar ist.

Figur 6 illustriert erneut den Haltearm 1, welcher bereits mit Bezug auf die Figuren 4 und 5 beschrieben worden ist. Gemäß diesem Ausführungsbeispiel und alternativ zu der beanspruchten Erfindung weist der Haltearm 1 eine kontaktlose Assistenz-Bedieneinrichtung 280 in Form einer Kamera mit integriertem Tiefensensor auf.

In Figur 6 ist der Haltearm 1 in ein System eingebunden dargestellt. An dem distalen Ende 4 ist mittels der Schnittstelle 8 ein chirurgisches mechatronisches Assistenzsystem 200 angeordnet, welches mit der Schnittstelle 8 über eine Schnittstelle 201 gekoppelt ist. Sowohl das chirurgische mechatronische Assistenzsystem 200 als auch die Schnittstelle 201 sind in Figur 6 nur schematisch dargestellt. Es soll verstanden werden, dass das chirurgische mechatronische Assistenzsystem 200 beispielsweise als Endoskop oder Laparoskop oder dergleichen ausgebildet sein kann. Das Assistenzsystem 200 weist einen Arbeitsabschnitt 202 auf, der beispielsweise die Spitze des Endoskops sein kann. An dem proximalen Ende 2 ist der Haltearm 1 gemäß Figur 6 über die mechanische Schnittstelle 7 mit einer Basis 204 gekoppelt. Die Basis 204 ist hier ebenfalls nur schematisch dargestellt. Sie kann beispielsweise als eine Normschiene eines Operationstisches ausgebildet sein.

Die erste Schnittstelle 6 ist gemäß diesem Ausführungsbeispiel mit einer externen Steuereinheit 206 gekoppelt. Dazu ist die Schnittstelle 6 mittels eines Kabels 208 mit der externen Steuereinheit 206 verbunden. Die externe Steuereinheit 206 ist gemäß diesem Ausführungsbeispiel als OP-System ausgebildet, welches beispielsweise einen herkömmlichen Computer aufweist sowie eine Eingabe-Ausgabe-Schnittstelle zur Bedienung des OP-Systems. Das OP-System weist vorzugsweise Software-Komponenten auf, die dazu ausgebildet sind, Daten, die von dem Haltearm 1 an der Schnittstellt 6 übergeben werden, zu speichern und zu verarbeiten.

Je nach Ausgestaltung der Schnittstelle 6 kann auch vorgesehen sein, dass diese drahtlos mit dem OP-System 206 kommuniziert, beispielsweise über Bluetooth^{®}, Wi-Fi^{®} oder Ähnliches.

Figur 7 zeigt eine Ausführungsform eines Armsegments, wobei hier das vierte Armsegment 18 dargestellt ist. Es soll erkannt werden, dass auch die anderen Armsegmente 10, 12, 14, 16, 20, 22 ebenso ausgebildet sein können. Im Inneren weist der Armsegmentkörper 90 einen Hohlraum 92 auf, in dem verschiedene Elemente, wie etwa die Bremse 70, angeordnet sind. Schematisch dargestellt in den Figuren 4 und 5 sind die Gelenke 19, 21 sowie die beiden Schwenkachsen A₄, A₅ der Gelenke 19, 21, die jeweils mit dem Haltearmsegment 18 zusammenwirken. Dem Haltearmsegment 18 ist das Gelenk 19 zugeordnet. Der Armsegmentkörper 90 weist eine äußere Oberfläche 93 auf, die im Wesentlichen zylindrisch ausgebildet ist. Der Armsegmentkörper 90 ist beispielsweise aus einem Metall wie insbesondere Aluminium oder Titan einer Legierung auf Basis von Aluminium oder Titan, oder einem Faserverbundwerkstoff, wie etwas GFK oder CFK, gebildet und vorzugsweise in Leichtbauweise ausgelegt.

Das Armsegment 18 weist gemäß Figur 7 eine Assistenz-Bedieneinrichtung 280 mit einem als berührungsempfindliches Sensorfeld ausgebildeten Kontaktmittel 238 auf, das Über eine Datenschnittstelle 240 am Bussystem 76 des Haltearms 1 angekoppelt ist. Es soll verstanden werden, dass das berührungsempfindliche Sensorfeld eine Alternative zu dem erfindungsgemäßen Steuerungsring darstellt.

Das Armsegment 18 weist gemäß Figur 7 ein Kontaktmittel 38 der Arm-Bedienungseinrichtung auf, welches Teil der Arm-Bedieneinrichtung 28 ist (vgl. Fig. 4, 5 und 6). Das Kontaktmittel 38 weist zwei Kontaktmittelelemente 38a, 38b auf, die als berührungsempfindliche Sensoren ausgebildet sind und flächenbündig in der äußeren Oberfläche 93 des Armsegments 18 angeordnet sind. Die beiden Kontaktmittelelemente 38a, 38b sind im Wesentlichen gegenüberliegend bezogen auf die Achse A₅ angeordnet, sodass ein Bediener beim Ergreifen des Armsegments 18 in Kontakt mit beiden Kontaktmittelelementen 38a, 38b kommt, wie dies oben beschrieben ist.

Die beiden Kontaktmittelelemente 38a, 38b sind mittels Leitungen 94a, 94b mit dem Bussystem 76 gekoppelt. Über das Bussystem 76 sind die Kontaktmittelelemente 38a, 38b mit der elektronischen Steuereinheit 74 gekoppelt und über diese wiederum mit der Bremse 70, sodass die Bremse 70 durch die Arm-Bedieneinrichtung 28 freigegeben wird, wenn ein Bediener mit den Kontaktmittelelementen 38a, 38b in Kontakt kommt.

Neben dem Bussystem 76 sind innerhalb des Armsegmentkörpers 90 ein Energieübertragungssystem 78 sowie ein Kabelkanal 80 und ein Arbeitskanal 82 angeordnet. Mittels des Energieübertragungssystems 78 sind die Kontaktmittelelemente 38a, 38b sowie die Bremse 70 an eine Energieversorgung angeschlossen.

Alternativ oder zusätzlich ist in jedem Armsegment ein Elektronikmodul 96 angeordnet, welches über eine Leitung 96a mit dem Bussystem 76 gekoppelt ist. In einem solchen Fall wirken die Kontaktmittelelemente 38a, 38b, welche über die Leitung 94a, 94b mit dem Datenbussystem 76 verbunden sind, nur mit dem Elektronikmodul 96 zusammen, welches den durch die Kontaktmittelelemente 38a, 38b erfassten Kontakt in ein Stellsignal für die Bremse 70 umwandelt und dieses Stellsignal über das Bussystem 76 an die Bremse 70 sendet zum Freigeben des Gelenks 19. Ist in jedem Armsegment ein derartiges Elektronikmodul 96 angeordnet, ist ein im Wesentlichen modularer Aufbau des Haltearms 1 realisiert, und die einzelnen Armsegmente 10 bis 22 sind unabhängig von der elektronischen Steuereinheit 74, welche im proximalen Armsegment 10 angeordnet ist.

Der Kabelkanal 80 dient dazu, Kabel, die vom proximalen Ende 2 bis zum distalen Ende 4 verlaufen, um insbesondere die Schnittstelle 8 zu versorgen, führen zu können. Der Arbeitskanal 82 dient dazu, Schläuche oder Lichtleiter und dergleichen aufzunehmen, die je nach Art des Manipulators, der an der Schnittstelle 8 angeordnet ist, benötigt werden. Ist beispielsweise an der Schnittstelle 8 ein Endoskop angeordnet, wird vorzugsweise durch den Arbeitskanal 82 ein Lichtleiter geführt, der ein Bild, welches eine Endoskopkamera aufnimmt, übertragen kann. Der Arbeitskanal 82 dient demnach dazu, je nach Anwendungsgebiet entsprechende Übertragungsmittel aufzunehmen.

Weiterhin ist in dem Armsegment 18 ein Sensor 98 angeordnet. Vorzugsweise ist in jedem Armsegment 10 bis 22 ein Sensor angeordnet, und es soll verstanden werden, dass die Sensoren in den Armsegmenten 10, 12, 14, 16, 20 und 22 ebenso ausgebildet sein können wie der Sensor 98 im Armsegment 18. Der Sensor 98 ist vorzugsweise als Beschleunigungssensor ausgebildet. Indem in jedem Armsegment ein solcher Beschleunigungssensor vorgesehen ist, ist es möglich, zu jeder Zeit die Pose des Haltearms 1 zu bestimmen. Dazu ist der Sensor 98 über eine Leitung 98a mit dem Datenbussystem 76 gekoppelt, sodass die von dem Sensor 98 erfassten Daten an die elektronische Steuereinheit 74 übertragen werden, welche dann aus allen Sensordaten aus allen Armsegmenten die Pose des Haltearms 1 bestimmt. Ferner ist durch das Vorsehen eines derartigen Sensors 98 auch die absolute und relative Position eines an der Schnittstelle 8 angeordneten Endeffektors beziehungsweise Manipulators bestimmbar. Auch ist es möglich, wenn der Haltearm 1 an einem Operationstisch angeordnet ist, eine Bewegung dieses Operationstischs zu erkennen. Erfassen alle Sensoren in allen Armsegmenten eine Bewegung in dieselbe Richtung, ist dies ein Indikator dafür, dass der gesamte Haltearm 1 unter Beibehaltung seiner Pose bewegt wurde, beispielsweise dadurch, dass der Operationstisch oder eine Operationstischplatte relativ zu einer Säule der Operationstisches rotiert oder verschoben wurde. Auch eine solche Bewegung ist mittels der Sensoren 98 erfassbar. Ferner können äußere Impulse, wie etwa Stöße auf den Haltearm 1, erfasst werden.

Gemäß Figur 7 ist in dem Armsegment 18 eine der Assistenz-Bedieneinrichtung 280 zugeordnete Verarbeitungseinheit 100 vorgesehen, die mit dem Datenbussystem 76 über die Leitung 100a gekoppelt ist.

Figur 8 zeigt ein erfindungsgemäßes Verfahren 1000 zum Steuern eines an einen Haltearm für medizinische Zwecke gekoppelten chirurgischen mechatronischen Assistenzsystems (siehe Figur 1 bis 6).

In einem ersten Schritt 1002 wird eine auf ein Steuern des Assistenzsystems gerichtete Bedienaktion eines Bedieners mittels einer an dem Haltearm angeordneten Bedieneinrichtung erfasst.

In einem zweiten Schritt 1004, der dem Verständnis dient, erfolgt ein Auslesen 1004 von den Haltearm betreffenden Sensordaten und Arbeitsraumdaten über die Übertragungseinrichtung.

In einem dritten Schritt 1006, der dem Verständnis dient, erfolgt ein Umwandeln der erfassten Bedienaktion in eine Datenstruktur unter Berücksichtigung der den Haltearm betreffenden Sensordaten und ein Erzeugen des Anforderungssignals aus der Datenstruktur.

Danach, in einem vierten Schritt 1008, der dem Verständnis dient, erfolgt ein Ermitteln ob das chirurgische mechatronische Assistenzsystem bei Ansteuerung gemäß des Anforderungssignals innerhalb eines zulässigen Arbeitsraums verbleiben würde.

In einem fünften Schritt 1010, der dem Verständnis dient, erfolgt ein Übertragen des die Bedienaktion repräsentierenden Anforderungssignals an die Assistenz-Schnittstelle zwecks Steuerung des chirurgischen mechatronischen Assistenzsystems.

Das Verfahren wird bevorzugt mit einem Haltearm 1 gemäß einer der vorstehend beschriebenen bevorzugten Ausführungsbeispiele des Haltearms 1 (Fig. 1 bis 7) ausgeführt.

## Patentansprüche

1. Haltearm (1) für medizinische Zwecke, insbesondere zum Halten eines mechatronischen Assistenzsystems (200), mit
- einem proximalen Ende (2) zum Befestigen des Haltearms (1) an einer Basis (204) und einem distalen Ende (4) zum Aufnehmen des chirurgischen mechatronischen Assistenzsystems (200);
- einer Basis-Schnittstelle (6) an dem proximalen Ende (2) zum Verbinden des Haltearms (1) mit einer externen Steuereinheit (206) zum Übertragen von Signalen an den und von dem Haltearm (1);
- einer Assistenz-Schnittstelle (8) an dem distalen Ende (4) zum Koppeln des Haltearms (1) mit dem Assistenzsystem (200) zum Steuern des Assistenzsystems (200);
- einer Übertragungseinrichtung (76, 78), welche innerhalb des Haltearms (1) angeordnet ist und die Assistenz-Schnittstelle (6) mit der Basis-Schnittstelle (8) zum Übertragen von Signalen zwischen den Schnittstellen (6, 8) verbindet;
- einer Assistenz-Bedieneinrichtung (280), die dazu eingerichtet ist, eine auf ein Steuern des Assistenzsystems (200) gerichtete Bedienaktion eines Bedieners zu erfassen und bedarfsweise ein Übertragen eines die Bedienaktion repräsentierenden Anforderungssignals (m) an die Assistenz-Schnittstelle (8) zwecks Steuerung des chirurgischen mechatronischen Assistenzsystems (200) zu bewirken; und
- einer der Assistenz-Bedieneinrichtung (280) zugeordneten Verarbeitungseinheit (100), die dazu eingerichtet ist die Bedienaktion in eine Datenstruktur umzuwandeln, wobei die Datenstruktur einen 3D-Bewegungsvektor (r) und einen Geschwindigkeitswert (v) umfasst,
**dadurch gekennzeichnet, dass** die Assistenz-Bedieneinrichtung (280) wenigstens einen Steuerungsring (29) aufweist, der um wenigstens ein Armsegment (20) herum umfänglich und/oder axial beweglich angeordnet ist, wobei wenn ein Bediener den Steuerungsring (29) um eine Längsachse (L) rotiert, dies durch die Verarbeitungseinheit (100) erkannt und ein entsprechendes Anforderungssignal, das eine Rotationsrichtung, einen Rotationswinkel, eine Rotationsgeschwindigkeit und/oder eine Rotationsbeschleunigung repräsentieren kann, über die Übertragungseinrichtung (76) an das chirurgische mechatronische Assistenzsystem (200) gesendet wird.

2. Haltearm nach Anspruch 1, wobei die Verarbeitungseinheit (100) zumindest zeitweise mit der Übertragungseinrichtung gekoppelt ist und dazu eingerichtet ist, den Haltearm (1) betreffende Sensordaten (SI1, SI2, SIN) und/oder Arbeitsraumdaten aus der Übertragungseinrichtung auszulesen.

3. Haltearm nach Anspruch 2, wobei die Verarbeitungseinheit (100) dazu eingerichtet ist, bei der Umwandlung der Bedienaktion in die Datenstruktur die den Haltearm betreffenden Sensordaten (SI1, SI2, SIN) zu berücksichtigen.

4. Haltearm nach einem der vorstehenden Ansprüche, wobei die Verarbeitungseinheit (100) dazu eingerichtet ist, die Datenstruktur in das Anforderungssignal umzuwandeln.

5. Haltearm nach einem der vorstehenden Ansprüche, wobei die Verarbeitungseinheit (100) dazu eingerichtet ist, zu ermitteln, ob das chirurgische mechatronische Assistenzsystem (200) bei Ansteuerung gemäß dem Anforderungssignal innerhalb eines zulässigen Arbeitsraums verbleiben würde, und wobei die Verarbeitungseinheit (100) dazu eingerichtet ist ein Übertragen des Anforderungssignals an das chirurgische mechatronische Assistenzsystem (200) nur zu bewirken, wenn das chirurgische mechatronische Assistenzsystem (200) bei Ansteuerung gemäß dem Anforderungssignal innerhalb eines zulässigen Arbeitsraum verbleiben würde.

6. Haltearm nach einem der vorangehenden Ansprüche, mit zwei oder mehr Armsegmenten (10, 12, 14, 16, 18, 20, 22) und zwei oder mehr Gelenken (11, 13, 15, 17, 19, 21, 23), mittels denen die Armsegmente (10, 12, 14, 16, 18, 20, 22) gelenkig miteinander verbunden sind, wobei zusätzlich zu der Assistenz-Bedieneinrichtung (280) eine Arm-Bedieneinrichtung (28) vorgesehen ist mittels derer wenigstens ein Gelenk, bevorzugt jedes Gelenk (11, 13, 15, 17, 19, 21, 23) freigebbar und arretierbar ist, so dass der Haltearm in eine gewünschte Pose bringbar ist, wobei die Arm-Bedieneinrichtung (28) bevorzugt dazu eingerichtet ist bei Kontakt zwischen einer Bedienperson und einen der zwei oder mehr Armsegmente (10, 12, 14, 16, 18, 20, 22) das zugeordnete Gelenk (11, 13, 15, 17, 19, 21, 23) freizugeben.

7. Verfahren (1000) zum Steuern eines an einen Haltearm für medizinische Zwecke nach einem der vorstehenden Ansprüche gekoppelten chirurgischen mechatronischen Assistenzsystems mit den Schritten:
- Erfassen (1002) einer auf ein Steuern des Assistenzsystems gerichteten Bedienaktion eines Bedieners mittels der an dem Haltearm (1) angeordneten Assistenz-Bedieneinrichtung (280), die den wenigstens einen Steuerungsring (29) aufweist, der um wenigstens ein Armsegment (20) herum umfänglich und/oder axial beweglich angeordnet ist;
- bedarfsweises (1010) Übertragen eines die Bedienaktion repräsentierenden Anforderungssignals, das eine Rotationsrichtung, einen Rotationswinkel, eine Rotationsgeschwindigkeit und/oder eine Rotationsbeschleunigung repräsentieren kann, an die Assistenz-Schnittstelle (8) zwecks Steuerung des chirurgischen mechatronischen Assistenzsystems;
- Umwandeln der erfassten Bedienaktion in eine Datenstruktur, wobei die Datenstruktur bevorzugt einen 3D-Bewegungsvektor und einen Geschwindigkeitswert umfasst; und
- Erzeugen des Anforderungssignals aus der Datenstruktur.

## Claims

1. A holding arm (1) for medical purposes, in particular for holding a surgical mechatronic assistance system (200), comprising
- a proximal end (2) for attaching the holding arm (1) to a base (204) and a distal end (4) for receiving the surgical mechatronic assistance system (200);
- a base interface (6) at the proximal end (2) for connecting the holding arm (1) to an external control unit (206) for transmitting signals to and from the holding arm (1);
- an assistance interface (8) at the distal end (4) for coupling the holding arm (1) to the assistance system (200) in order to control the assistance system (200);
- a transmission means (76, 78) which is arranged inside the holding arm (1) and which connects the assistance interface (6) to the base interface (8) in order to transmit signals between the interfaces (6, 8);
- an assistance operating device (280) designed to detect an operator action by an operator which is directed at controlling the assistance system (200) and if necessary to cause a request signal representing the operator action (m) to be transmitted to the assistance interface (8) for the purpose of controlling the surgical mechatronic assistance system (200); and
- a processor unit (100) assigned to the assistance operating device (280) and configured to translate the operator action into a data structure, wherein the data structure comprises a 3D motion vector (r) and a velocity value (v),
**characterized in that** the assistance operating device (280) comprises at least one control ring (29) which is arranged around the circumference of at least one arm segment (20) and/or is axially mobile, wherein when an operator rotates the control ring (29) around a longitudinal axis (L), this is detected by the processor unit (100) and a corresponding request signal which can represent a direction of rotation, an angle of rotation, a rotational speed and/or a rotational acceleration, is sent via the transmission means (76) to the mechatronic assistance system (200).

2. The holding arm according to claim 1, wherein the processor unit (100) is coupled at least temporarily to the transmission means and is configured to fetch sensor data (SI1, SI2, SIN) and/or workspace data relating to the holding arm (1) from the transmission means.

3. The holding arm according to claim 2, wherein the processor unit (100) is configured to take the sensor data (SI1, SI2, SIN) relating to the holding arm into account when translating the operator action into the data structure.

4. The holding arm according to any one of the preceding claims, wherein the processor unit (100) is configured to convert the data structure into the request signal.

5. The holding arm according to any one of the preceding claims, wherein the processor unit (100) is configured to detect whether the surgical mechatronic assistance system (200) would remain within a permitted workspace when driven in accordance with the request signal, and wherein the processor unit (100) is configured to cause the request signal to be transmitted to the surgical mechatronic assistance system (200) only if the surgical mechatronic assistance system (200) would remain within a permitted workspace when driven in accordance with the request signal.

6. The holding arm according to any one of the preceding claims, comprising two or more arm segments (10, 12, 14, 16, 18, 20, 22) and two or more joints (11, 13, 15, 17, 19, 21, 23), by means of which the arm segments (10, 12, 14, 16, 18, 20, 22) are connected to each other by joints, wherein in addition to the assistance operating device (280) there is also provided an arm operating device (28) by means of which at least one joint and preferably every joint (11, 13, 15, 17, 19, 21, 23) is releasable and lockable such that the holding arm can be brought into a desired pose, the arm operating device (28) preferably being designed to release the associated joint (11, 13, 15, 17, 19, 21, 23) upon contact between an operator and one of the two or more arm segments (10, 12, 14, 16, 18, 20, 22).

7. A method (1000) for controlling a surgical mechatronic assistance system coupled to a holding arm for medical purposes according to one of the preceding claims, said method comprising the steps of:
- detecting (1002) an operator action by an operator which is directed at controlling the assistance system, by means of an operating device (280) arranged on the holding arm (1), the operating device comprising the at least one control ring (29) which is arranged around the circumference of at least one arm segment (20) and/or is axially mobile;
- transmitting (1010), if necessary, a request signal representing the operator action, wherein the request signal can represent a direction of rotation, an angle of rotation, a rotational speed and/or a rotational acceleration, to the assistance interface for the purpose of controlling the surgical mechatronic assistance system;
- translating the detected operator action into a data structure, wherein the data structure preferably comprises a 3D motion vector and/or a velocity value; and
- generating the request signal from the data structure.

## Revendications

1. Bras de maintien (1) à usage médical, en particulier pour le maintien d'un système d'assistance mécatronique (200), avec
- une extrémité proximale (2) pour fixer le bras de maintien (1) à une base (204) et une extrémité distale (4) pour recevoir le système d'assistance mécatronique chirurgical (200);
- une interface de base (6) à l'extrémité proximale (2) pour connecter le bras de maintien (1) à une unité de commande externe (206) pour transmettre des signaux vers et depuis le bras de maintien (1);
- une interface d'assistance (8) à l'extrémité distale (4) pour coupler le bras de maintien (1) au système d'assistance (200) pour commander le système d'assistance (200);
- un dispositif de transmission (76, 78), lequel est disposé à l'intérieur du bras de maintien (1) et connecte l'interface d'assistance (6) à l'interface de base (8) pour la transmission de signaux entre les interfaces (6, 8);
- un dispositif de commande d'assistance (280), qui est configuré pour détecter une action de commande d'un opérateur dirigée pour commander le système d'assistance (200) et, si nécessaire, pour provoquer une transmission d'un signal de demande (m) représentant l'action de commande à l'interface d'assistance (8) afin de commander le système d'assistance mécatronique chirurgical (200); et
- une unité de traitement (100) associée au dispositif de commande d'assistance (280), qui est configurée pour convertir l'action de commande en une structure de données, dans lequel la structure de données comprend un vecteur de mouvement 3D (r) et une valeur de vitesse (v),
**caractérisé en ce que** le dispositif de commande d'assistance (280) présente au moins une bague de commande (29) qui est disposée de manière mobile sur le pourtour et/ou axialement autour d'au moins un segment de bras (20), dans lequel lorsqu'un opérateur fait tourner la bague de commande (29) autour d'un axe longitudinal (L), ceci est détecté par l'unité de traitement (100) et un signal de demande correspondant, qui peut représenter une direction de rotation, un angle de rotation, une vitesse de rotation et/ou une accélération de rotation, est envoyé au système d'assistance mécatronique chirurgical (200) par l'intermédiaire du dispositif de transmission (76).

2. Bras de maintien selon la revendication 1, dans lequel l'unité de traitement (100) est au moins temporairement couplée au dispositif de transmission et est configurée pour lire des données de capteur (SI1, SI2, SIN) et/ou des données d'espace de travail concernant le bras de maintien (1) à partir du dispositif de transmission.

3. Bras de maintien selon la revendication 2, dans lequel l'unité de traitement (100) est configurée pour prendre en compte les données de capteur (SI1, SI2, SIN) concernant le bras de maintien lors de la conversion de l'action de commande dans la structure de données.

4. Bras de maintien selon l'une quelconque des revendications précédentes, dans lequel l'unité de traitement (100) est configurée pour convertir la structure de données en signal de demande.

5. Bras de maintien selon l'une quelconque des revendications précédentes, dans lequel l'unité de traitement (100) est configurée pour déterminer si le système d'assistance mécatronique chirurgical (200) resterait dans un espace de travail admissible en cas de commande selon le signal de demande, et dans lequel l'unité de traitement (100) est configurée pour provoquer une transmission du signal de demande au système d'assistance mécatronique chirurgical (200) uniquement si le système d'assistance mécatronique chirurgical (200) reste dans un espace de travail admissible en cas de commande selon le signal de demande.

6. Bras de maintien selon l'une quelconque des revendications précédentes, avec deux ou plusieurs segments de bras (10, 12, 14, 16, 18, 20, 22) et deux ou plusieurs articulations (11, 13, 15, 17, 19, 21, 23), au moyen desquels les segments de bras (10, 12, 14, 16, 18, 20, 22) sont connectés de manière articulée, dans lequel un dispositif de commande de bras (28) est prévu en plus du dispositif de commande d'assistance (280), au moyen duquel au moins une articulation, de préférence chaque articulation (11, 13, 15, 17, 19, 21, 23), peut être libérée et bloquée, de sorte que le bras de maintien peut être amené dans une position souhaitée, dans lequel le dispositif de commande de bras (28) est de préférence configuré pour libérer l'articulation associée (11, 13, 15, 17, 19, 21, 23) en cas de contact entre un opérateur et un des deux ou plusieurs segments de bras (10, 12, 14, 16, 18, 20, 22).

7. Procédé (1000) de commande d'un système d'assistance mécatronique chirurgical couplé à un bras de maintien à usage médical selon l'une quelconque des revendications précédentes avec les étapes consistant à :
- détecter (1002) une action de commande d'un opérateur dirigée pour commander le système d'assistance au moyen du dispositif de commande d'assistance (280) disposé sur le bras de maintien (1), qui présente l'au moins une bague de commande (29), qui est disposée de manière mobile sur le pourtour et/ou axialement autour d'au moins un segment de bras (20);
- transmettre, au besoin (1010), un signal de demande représentant l'action de commande, qui peut représenter une direction de rotation, un angle de rotation, une vitesse de rotation et/ou une accélération de rotation, à l'interface d'assistance (8) afin de commander le système d'assistance mécatronique chirurgical;
- convertir l'action de commande détectée en une structure de données, dans lequel la structure de données comprend de préférence un vecteur de mouvement 3D et une valeur de vitesse; et
- générer le signal de demande à partir de la structure de données.
